# EUROPEAN PATENT SPECIFICATION

(11) **EP 1 104 772 B1**
(45) Date of publication and mention of the grant of the patent: **20.10.2004**
(21) Application number: 99933198.6
(22) Date of filing: 30.07.1999
(51) Int. Cl.: C07K 16/12, C12P 21/08, C12N 15/00, C12Q 1/04, G01N 33/53

(54) **ANTI-L7/L12 ANTIBODY FOR DETECTING BACTERIA**
ANTI-L7/L12-ANTIKÖRPER ZUM NACHWEIS VON BAKTERIEN
ANTI-L7/L12 ANTICORPS POUR LA DETECTION DE BACTERIES

(30) Priority: 31.07.1998 JP 23020498
(43) Date of publication of application: 06.06.2001
(73) Proprietor: Asahi Kasei Kabushiki Kaisha, Osaka-shi, Osaka 530-8205 (JP)
(72) Inventor: MATSUYAMA, Kenji, Boulder, CO 80301 (US); SHIRAI, Takashi, Sunto-gun, Shizuoka 416-0943 (JP); ETOH, Takashi, Fuji-shi, Shizuoka 417-0801 (JP)
(74) Representative: Forstmeyer, Dietmar, Dr. rer. nat., Dipl.-Chem.
(86) International application number: PCT/JP1999/004122
(87) International publication number: WO 2000/006603

(56) References cited:
- EP-A- 0 543 091
- JP-A- 1 500 083
- SOMMER A ET AL: "PREPARATION AND CHARACTERIZATION OF 2 MONOCLONAL ANTIBODIES AGAINST DIFFERENT EPITOPES IN ESCHERICHIA-COLI RIBOSOMAL PROTEIN L-7-L-12" JOURNAL OF BIOLOGICAL CHEMISTRY, vol. 260, no. 11, 1985, pages 6522-6527, XP001055585 ISSN: 0021-9258
- COOPER M D ET AL: "IMMUNOGENICITY OF RIBOSOMAL PREPARATIONS FROM NEISSERIA-GONORRHOEAE" INFECTION AND IMMUNITY, vol. 28, no. 1, 1980, pages 92-100, XP001057146 ISSN: 0019-9567
- LEVY R ET AL: "DETECTION OF ANTIBODIES AGAINST CANDIDA-ALBICANS RIBOSOMES BY THE ENZYME-LINKED IMMUNOSORBENT ASSAY ELISA" MYCOPATHOLOGIA, vol. 87, no. 3, 1984, pages 167-170, XP001068537 ISSN: 0301-486X
- JAN KOLBERG ET AL: 'Monoclonal Antibodies Against Streptococcus Pneumoniae Detect Epitopes on Eubacterial Ribosomal Protein L7/L12 and on Streptococcal Elongation Factor Ts' MICROBIOLOGY vol. 143, no. 1, 1997, pages 55 - 61, XP002925389
- SERGYL LAFONT ET AL: 'Induction of Murine B Cell Proliferation and Immunoglobulin Synthesis by Some Bacterial Ribosomes' MICROBIOL. IMMUNOL., vol. 32, no. 10, 1988, pages 1043 - 1058, XP002925390
- KITA E. ET AL: 'Analysis of Immune Responses in Genital Tracts of Mice Immunised with Purified Ribosomal Fractions Neisseria-Gonorrhoeae' BRITISH JOURNAL OF VENEREAL DISEASES vol. 60, no. 4, 1984, pages 219 - 225, XP000905316

## Description

### DESCRIPTION OF BACKGROUND ART

### Field of the Invention

The present invention pertains to antibodies useful in the detection of various bacteria, and a method of detecting bacteria, reagent kits for detection of bacteria that use said antibodies, and a method for preparing specific antibodies for detecting bacteria.

Moreover, the present invention is valuable to the drug industry, particularly for diagnostic medicine of bacterial infections.

### Prior Art

Diagnosis of microbial infection is confirmed by detection of the causative pathogen from the infected area or by detection of antibodies to the causative pathogen in serum and body fluids. Detection of the causative pathogen is particularly important in the sense that it makes quick treatment to the patient possible.

Detection of the causative pathogen of infections can be generally classified as cultivation and identification methods, whereby the causative pathogen is separated and cultivated and then identified based on its biochemical properties; genetic diagnosis, whereby amplification by PCR, is performed based on specific genes of the causative pathogen and thus the causative pathogen is detected; or immunological methods, whereby the causative pathogen is detected using a specific reaction of antibody with surface antigen marker of the causative pathogen. However, it takes time to obtain results by cultivation and identification methods or genetic diagnosis methods. Therefore, diagnosis by immunological methods is commonly used because the causative pathogen can be detected within a short time with high sensitivity and the patient can be quickly and appropriately treated.

Depending on the bacterial species, a variety of marker antigens and antibodies can be used alone or, in combination to detect the causative pathogen of infections by conventional immunological methods.

For instance, it is known that lipopolysaccharide (LPS), which is a genus-specific antigen of *Chlamydia,* is present as an antigen determinant (Stephens, R., et al.: *J. Immunol*., 128:1083-89, 1982, Caldwell, M.D.: *Inf. Immun*., 44:306-14, 1984), and antibodies to LPS are used as the reagent antibody in a variety of diagnostic kits, particularly for the detection of *Chlamydia trachomatis.*

Moreover, Ellena M. Peterson et al., (Infection and Immunity, 59(11), 4147-4153, 1991) and Byron E. Batteiger et al., (Infection and Immunity, 53(3), 530-533, 1986) have reported monoclonal antibodies to major outer membrane protein (MOMP) of the genus *Chlamydia* respectively.

Publication of unexamined Japanese patent application No. 298/1988 discusses an immunodetection method based on western blot method that uses a monoclonal antibody to an approximately 43 kilo Dalton membrane protein antigen of *Mycoplasma pneumoniae.*

Moreover, a method of preparing monoclonal antibody to *Haemophilus influenzae* and a diagnostic method that uses said antibody are presented in Publication of unexamined Japanese patent application No. 148859/1987 (Japanese Patent No. 64065/1994).

Proteins of approximately 20 kilo Dalton isolated from the sodium cholate extract of the outer membrane vesicle of *Neisseria gonorrhoeae* strain BS4 (NCTC 11922) are documented and preparation of hybridomas using said substance is disclosed in British Patent Application No. 2,172,704. Moreover, European Patent Document no., EP 419238 A1 describes preparation of a monoclonal antibody, which can bind to a protein of approximately 14 kilo Dalton prepared by using *Neisseria gonorrhoeae* as an immunogen and a method for the preparation of such a monoclonal antibody. Additionally, a method of detecting the same *Neisseria gonorrhoeae* using monoclonal antibody to lipopolysaccharide (LPS) is mentioned in Canadian Patent Application No. 1,220,147.

However, there are problems with said antibodies and detection methods based on those antibodies in that species specificity to microorganisms is insufficient for proper diagnosis. The antibodies do not detect all serum types as plural surface antigens present within one species.

Marker antigens used in these prior arts are not standardized so that microorganisms can be detected using a same functional molecule (for instance, protein, LPS or surface polysaccharide component with the same function) which is generally present in cells of various microorganisms and which changes during the course of evolution of the microorganisms as a marker, and an immunodiagnostic method based on the concept of detecting the difference in antigenicity between bacterial species using one molecule as a standard is not known.

### DISCLOSURE OF THE INVENTION

In the context of this application the term "microorganism(s)" is to be understood as equal the term "bacteria".

The present invention strives to present antibodies to the same molecule for different microorganisms as a standard marker antigen to make ideal microorganism detection and immunodiagnosis possible, particularly antibodies to the molecular segment in the same intracellular functional component molecule for all microorganisms to be detected that changes during the course of evolution of microorganisms, a method of detecting microorganisms, which is species specific and can cover almost all serum types, a reagent kit for detection of microorganisms that use said antibody, and a method for preparing a specific antibody used for detection of microorganisms.

The present inventors discovered a protein with the same function, preserved in all microorganisms as a useful antigen protein. Usually, it is expected that a structural change of such a protein is extremely small. However, surprisingly it was found that the antigen epitope(s) of this protein has specificity to certain species or genus of microorganisms, and that the antibody for this protein not only has potentialities of being used for specifically identifying species or genus of microorganisms, but also is capable of detecting all serotypes of the target microorganisms.

The inventors focused on intracellular molecule that are present in all microbial cells and differ between microorganisms in terms of its amino acid sequence, particularly ribosomal protein L7/L12, which is a member of ribosomal proteins. Ribosomal protein L7/L12 is a protein with a molecular weight of approximately 13 kilo Daltons and is known to exist as a ribosomal protein essential in protein synthesis. Progress has been made in understanding the complete amino acid sequence of the protein from several microorganisms including particularly *Escherichia coli and Baccillus subtilus,* and 50 % to 65 % homology of the amino acid sequence between the microorganisms has been confirmed.

The inventors focused on the fact that even though there are similarities in said molecule between different microorganisms, this molecule also has a structural segment that is unique to each microorganism and discovered that it is possible to detect various microorganisms with species specificity and to detect all serotypes within the same species by using antibody to the protein. As a result of attempting to develop a technology for immuno-diagnosis of microorganism species using antibody specific to, for instance, *Haemophilus influenzae*, *Streptococcus pneumoniae,* and *Neisseria gonorrhoeae*, the inventors completed the present invention upon discovering that antibody specific to said protein of each species of microorganisms can be obtained and species-specific detection of different bacteria is possible using said antibody.

The present invention relates to an antibody used for detecting bacteria, a method of detecting bacteria using the antibody, a reagent kit for detecting bacteria using the antibody, and a method for preparing specific antibodies for detecting bacteria.
1) Antibodies which are antibodies to ribosomal protein L7/L12 of bacteria and which react specifically with said bacteria.
2) The antibodies described in 1) above, where said bacteria are bacteria, which cause a sexually transmitted disease (STD).
3) The antibodies described in 1) above, where said bacteria are bacteria which cause respiratory tract infection.
4) The antibody described in 3) above, where the causative bacteria of respiratory tract infection are bacteria of *Haemophilus influenzae.*
5) The antibody described in 3) above, where the causative bacteria of respiratory tract infection are bacteria of *Streptococcus* *pneumoniae*.
6) The antibody described in 3) above, where the causative bacteria of respiratory tract infection are bacteria of Chlamydia pneumoniae.
7) The antibody described in 3) above, where the causative bacteria of respiratory tract infection are bacteria of Mycoplasma pneumoniae.
8) The antibody described in 2) above, where the causative bacteria of sexually transmitted diseases (STD) are bacteria of *Neisseria gonorrhoeae*.
9) The antibody described in 8) above, which is the antibody to ribosomal protein L7/L12 of *Neisseria gonorrhoeae* and which recognizes a continuous amino acid sequence moiety from 5 to 30 amino acids including the 115th alanine in the amino acid sequence of Sequence ID No. 22 of the Sequence Table.
10) A method of detecting bacteria, which is characterized by the fact that any antibody described in 1) to 9) above is used.
11) A reagent kit for detecting microorganisms, which is characterized by the fact that any antibody described in 1) to 9) above is used.
13) A method of preparing any antibody described in 1) to 9) above, characterized by the fact that ribosomal protein L7/L12 of microorganisms obtained by a gene manipulation procedure or by isolation from microorganisms, peptide moiety thereof, or a synthesized peptide corresponding to the peptide moiety is used as an immunogen.

The present invention will now be explained in detail.

Sequences No. 1 and No. 2 in the Sequence Table are the DNA sequence of the ribosomal protein L7/L12 gene of *Haemophilus influenzae* and corresponding amino acid sequence. Sequences No. 3 and No. 4 are the DNA sequence of the ribosomal protein L7/L12 gene of *Helicobacter pylori* and the corresponding amino acid sequence. Sequences No. 5 and No. 6 show the DNA sequence and the corresponding amino acid sequence of the ribosomal protein L7/L12 gene of *Streptococcus pneumoniae.* Sequences No. 7 and No. 8 show the DNA sequence and the corresponding amino acid sequence of the ribosomal protein L7/L12 gene of *Neisseria gonorrhoeae.* Sequences No. 9 and No. 10 show the DNA sequence and the corresponding amino acid sequence of the ribosomal protein L7/L12 gene of *Neisseria meningitidis.* Sequence No. 11 and Sequence No. 12 in the Sequence Table are the primers DNA for PCR used to acquire the ribosomal protein L7/L12 gene from *Haemophilus influenzae.* Sequences No. 13 and No. 14 in the Sequence Table are the primer DNA for PCR used to acquire the ribosomal protein L7/L12 gene from *Streptococcus pneumoniae.* Sequences No. 15 and No. 16 in the Sequence Table are the primer DNA for PCR used to acquire the ribosomal protein L7/L12 gene from *Neisseria gonorrhoeae.* Sequences No. 17 and No. 18 show the DNA sequence and the corresponding amino acid sequence of the ribosomal protein L7/L12 gene of *Haemophilus influenzae*. Sequences No. 19 and No. 20 show the DNA sequence and the corresponding amino acid sequence of the ribosomal protein L7/L12 gene of *Streptococcus pneumoniae.* Sequences No. 21 and No. 22 show the DNA sequence and the corresponding amino acid sequence of the ribosomal protein L7/L12 gene of Neisseria *gonorrhoeae.*

Furthermore, the left and right terminals of the amino acid sequences entered in the Sequence Table are amino group (referred to below as the N terminal) and carboxyl group terminals (referred to below as the C terminal), respectively, and the left terminal and right terminal of the base sequence is the 5' terminal and the 3' terminal, respectively.

Moreover, the series of biomolecular experiments of gene preparation mentioned in this text can be performed by methods entered in standard experimental manuals. "Molecular cloning: A laboratory manual," Cold Spring Harbor Laboratory Press, Sambrook, J. et al. (1989), is an example of the aforementioned standard experimental manual.

The term microorganism in the present invention refers to all species of bacteria, which bacteria in particular, pose a problem in terms of diagnosis of microbial infections

The term "antibody which reacts specifically with microorganisms" as used in the present invention means an antibody, which reacts specifically with a species or group of microorganisms. An antibody, which reacts specifically with a species of microorganisms, is particularly useful for the diagnosis of microbial infection diseases.

In the present invention, causative microorganisms of STD (sexually transmitted disease) include, but are not limited to, *Neisseria gonorrhoeae*, *Chlamydia trachomatis*, *Candida albicans*, *Treponema pallidum*, and *Ureaplasma urealyticum.*

In the present invention, causative microorganisms of respiratory tract infection include, but are not limited to, *Haemophilus influenzae*, *Streptococcus pneumoniae*, *Chlamydia pneumoniae*, *Mycoplasma pneumoniae*, *Klebsiella pneumoniae*, *Staphylococcus aureus*, *Pseudomonas aeruginosa*, *Streptococcus sp.* Group-A, *Mycobacterium tuberculosis*, *Legionella pneumophila*, and *Aspergillus spp*.

The term antibody in the present invention means a polyclonal antibody or monoclonal antibody that can be made using the entire length or only a partial peptide of said ribosomal protein. Although there are no special restrictions to the peptide length for making the antibody, the segment should be of the length characterizing the ribosomal protein L7/L12, and a peptide of 5 amino acids or longer, particularly 8 amino acids or longer, is preferred. Antiserum containing antibody (polyclonal antibody) that identifies ribosomal protein L7/L12 can be obtained by inoculating laboratory animals with adjuvant and a peptide or the full length protein as it is or when necessary, after being cross linked with a carrier protein such as KLH (keyhole-limpet hemocyanin) and BSA (bovine serum albumin) and recovering the serum. Moreover, the antibody can be used after it has been purified from the antiserum. The laboratory animals that are inoculated include sheep, horses, goats, rabbits, mice, rats, etc., and sheep, rabbits, etc., are particularly preferred for preparation of polyclonal antibody. Moreover, monoclonal antibody can also be obtained by conventional methods of making hybridoma cells, but mice are preferred in this case. The entire length of the said protein, or its partial peptide consisting amino acid residues of 5 or more, preferably 8 or more, residues that has been fused with GST (glutathione S-transferase), etc., can be purified and used as antigen, or it can be used as antigen without being purified. The antibody can also be the genetic recombination antibody expressed cellularly using immunoglobulin genes that have been separated by a variety of methods in manuals ("Antibodies: A Laboratory manual,"E. Harlow et al., Cold Spring Harbor Laboratory), cloning methods, etc.

Antibody to ribosomal protein L7/L12 that can be employed as the marker antigen of the present invention can be obtained by the following 3 methods, and other similar methods as well. The methods, however, are not limited to these.
a) The desired antibody can be acquired by synthesizing a peptide fragment consisting of 5-30 amino acids for microorganisms with a known ribosomal protein L7/L12 genetic sequence and amino acid sequence using the region least similar to the amino acid sequence of said protein of another microorganisms and making polyclonal antibody, or monoclonal antibody, using this peptide fragment as the immunogen.
   Moreover, it is possible to acquire the entire sequence of said gene by using a conventional genetic procedure, such as gene amplification by PCR with the DNA sequence at both terminals of said known genetic sequence as the probe, or hybridization using the sequence of a homologous segment as the template probe.
   Then, a fused gene with another protein gene is constructed and said fused gene is inserted into the host by conventional gene insertion methods using *Escherichia coli*, etc., as the host and expressed in large quantities. The desired protein antigen can then be acquired by purifying the expressed protein by affinity column methods with antibody to the protein that was used as the fusion protein. In such a case, even if antibody to the amino acid segment conserved within microorganisms is acquired, it does not coincide with the purpose of the present invention because the full length of ribosomal protein L7/L12 becomes the antigen. Consequently, hybridoma that produces monoclonal antibody to the antigen that has been obtained by this method is acquired by conventional methods and the desired antibody can be obtained by selecting a clone that produces antibody that will react only with the desired microorganisms.
b) First, since there is 50 to 60% homology between bacterial species in terms of their ribosomal protein L7/L12 amino acid sequence, it is possible to easily acquire said protein genes for microorganisms with unknown L7/L12 amino acid sequence by conventional genetic procedures, such as gene amplification of a specific sequence segment by PCR methods based on the sequence of the homologous segments of the amino acid sequence, or hybridization with the homologous segments as the template probe, using bacteria having a known ribosomal protein L7/L12 amino acid sequence.
   Then, a fused gene with another protein gene is constructed and said fused gene is inserted into the host by conventional gene insertion methods using *Escherichia coli*, etc., as the host and expressed in large quantities. The desired protein antigen can then be acquired by purifying the expressed protein by affinity column methods with antibody to the protein that was used as the fusion protein. In such a case, even if antibody to the amino acid segment conserved within microorganisms is acquired, it does not coincide with the purpose of the present invention because the full length of ribosomal protein L7/L12 becomes the antigen. Consequently, hybridoma that produces monoclonal antibody to the antigen that has been obtained by this method is acquired by conventional methods and the desired antibody can be obtained by selecting a clone that produces antibody that will react only with the desired microorganisms.
c) Ribosomal protein L7/L12 that has been purified to a high purity can also be obtained by another method, in the case where the amino acid sequence of the ribosomal protein L7/L12 is unknown whereby a peptide of 5 to 30 amino acids corresponding to the common sequence segment retained in the microorganisms is synthesized from the known amino acid sequence of the ribosomal protein L7/L12, and polyclonal antibody or monoclonal antibody to this peptide sequence is made by conventional methods. Then the highly purified Ribosomal protein L7/L12 from disrupted microorganisms is obtained by affinity column chromatography using said antibody.

If purity of the protein is insufficient, it can be purified by conventional methods, such as ion exchange chromatography, hydrophobic chromatography, gel filtration, etc., after which the eluted fraction of ribosomal protein L7/L12 is identified by western blotting, etc., using antibody that was made to obtain the pure fraction. The desired antibody can be obtained by acquiring hybridoma or polyclonal antibody by conventional methods using the pure ribosomal protein L7/L12 antigen that has been obtained and selecting hybridoma or polyclonal antibody that will react specifically with the desired bacteria, as in b).

The antibody of the present invention specific to a variety of microorganisms that has been obtained by the methods in a) through c), etc., can be used in a variety of immunoassay methods to obtain diagnostic reagent kits specific to a variety of microorganisms. For example, this antibody can be used in aggregation reactions where antibody is adsorbed on polystyrene latex particles, ELISA, which is a conventional technology performed in a microtiter plate, conventional immunochromatography methods, sandwich assay, whereby said antibody labeled with colored particles or particles that have coloring capability, or with enzyme or phosphor, and magnetic microparticles coated with capture antibody, etc., are used, etc.

The term detection methods for microorganism using antibody means detection methods that use conventional immunoassay such as aggregation reactions where antibody is adsorbed on polystyrene latex particles, ELISA, which is a conventional technology performed in a microtiter plate, conventional immunochromatography methods, sandwich assay, whereby said antibody labeled with colored particles or particles that have coloring capability, or with enzyme or phosphor, and magnetic microparticles coated with capture antibody, etc., are used, etc.

Moreover, the optical immunoassay (OIA) technology described in International Patent Application Japanese Laid-open (Toku-Hyou) No. 509565/1995, in which microorganisms are detected by an optical interference induced by an antibody reaction on the optical thin film which is formed by silicone or silicon nitride, is a useful detection method using an antibody.

Moreover, treatment with an extraction reagent that uses a variety of surfactants, beginning with Triton X-100 and Tween-20, enzyme treatment with an appropriate enzyme, such as protease, etc., known methods whereby the cell structure is disrupted, beginning with disruption of the microorganism by physical methods, can be used to extract the intracellular marker antigen from the necessary microorganism in said detection method. However, it is preferred that the extraction conditions be designed using a combination of surfactants, etc., so that the conditions are optimized for extraction of each microorganism with reagents.

Moreover, the term a reagent kit for detection of microorganisms using antibody means a reagent kit that uses said detection method.

For instance, in the case of obtaining the specific antibody to *Haemophilus influenzae*, which is of extreme diagnostic significance as a causative pathogen of pneumonia, bronchitis, meningitis, etc., the amino acid sequence and DNA sequence of ribosomal protein L7/L12 is entered in data bases, etc.

The amino acid and DNA sequence of ribosomal protein L7/L12 of *Haemophilus influenzae* are shown in "Sequences No. 1 and No. 2."

Consequently, in the case of this bacteria, it is possible to similarly compare the amino acid sequence of ribosomal protein L7/L12 with the same protein of, for instance, *Helicobacter pylori*, which is shown in "Sequence No. 3 and No. 4," and synthesize a peptide of 5 to 30 amino acids for the segment of low homology and make polyclonal antibody or monoclonal antibody specific to *Haemophilus influenzae* using this peptide.

In the case of a specific polyclonal antibody, it is preferred that IgG fraction be obtained by purification of the antiserum of immunized laboratory animals with a protein A column, etc., and affinity purification be performed with the synthetic peptide used in immunization of the laboratory animals.

Moreover, PCR primers based on the sequences of N-terminal and C-terminal, for example, the PCR primers shown in Sequences No. 11 and No. 12 in the Sequence Table, were designed from the DNA sequence of ribosomal protein L7/L12 of *Haemophilus influenzae.* Utilizing homology of the PCR primers, DNA fragments amplified by the PCR method or the like using genomic DNA which is extracted from cultivated *Haemophilus influenzae* can be acquired by a conventional method. The entire length of the gene for ribosomal protein L7/L12 of *Haemophilus influenzae* can be acquired by the analysis of the DNA sequence information of these fragments.

The ribosomal protein L7/L12 gene of *Haemophilus influenzae* thus acquired forms a fusion protein gene with, for instance, GST etc., and expression vector is constructed using the appropriate expression plasmid, *Escherichia coli* is transformed and large quantities of said protein can be expressed. A suitable amount of the transformed *Escherichia coli* is cultivated and the crushed bacterial fluid that is recovered is purified by an affinity column using GST to obtain the ribosomal protein L7/L12 and GST fusion protein of *Haemophilus influenzae*. It is also possible to acquire the target specific monoclonal antibody by establishing plural hybridomas using said protein as it is or GST moiety fragments cut from the protein by protease or the like, as an antigen protein and selecting the antibody which exhibits a specific response to *Haemophilus influenzae* bacteria, or a disrupt fluid of the bacteria, or ribosomal protein L7/L12 of *Haemophilus influenzae.*

Moreover, the amino acid sequence and the DNA sequence of ribosomal protein L7/L12 of *Streptococcus* *pneumoniae* which is also highly significant as a diagnostic agent for respiratory infection diseases as well as *Haemophilus influenzae*, are known from descriptions in data bases and the like. The amino acid and DNA sequences of ribosomal protein L7/L12 of *Streptococcus pneumoniae* are shown in Sequences No. 5 and No. 6 of sequence table.

It is therefore possible to acquire a polyclonal antibody or monoclonal antibody which is specific to *Streptococcus pneumoniae* by designing a PCR primer, the PCR primer shown by Sequence ID No. 13 or 14 in the Sequence Table, for example, based on the sequences of N-terminal and C-terminal of DNA sequence of Ribosomal Proteins L7/L12 of *Streptococcus pneumoniae* in the same manner as in the case of *Haemophilus influenzae*, and processing thereafter in the same manner as in the case of *Haemophilus influenzae*.

The hybridoma AMSP-2 which produces the monoclonal antibody specific to *Streptococcus pneumoniae* has been deposited with the National Institute of Bioscience and Human-Technology, the Agency of Industrial Science and Technology, the Ministry of International Trade and Industry, Japan, on July 28, 1999, with the deposition number FERM BP-6807.

Moreover, although the DNA and amino acid sequences of the ribosomal protein L7/L12 of, for instance, *Neisseria gonorrhoeae,* which is the causative pathogen of gonorrhea and has been shown to have diagnostic significance as a typical causative pathogen of STD, were unknown, a major part of the DNA sequence and amino acid sequence, which was determined by the Neisseria Gonorrhea Genome Project at Oklahoma University, USA, is disclosed on the Internet.

When part of the known DNA sequence of ribosomal protein L7/L12 was used to probe the existence of DNA fragments with a similar sequence, it was found that DNA sequence corresponding to the ribosomal protein L7/L12 gene is present and it was possible to obtain data on its entire DNA sequence. The entire base sequence and corresponding amino acid sequence of the ribosomal protein L7/L12 gene of this *Neisseria gonorrhoeae* are shown in Sequences No. 7 and No. 8 of the sequence table.

It is therefore possible to acquire the target antibody which is specific to *Neisseria gonorrhoeae* having the entire or partial Ribosomal Protein L7/L12 of *Neisseria gonorrhoeae* as an antigen by designing a PCR primer, the PCR primer shown by Sequence ID No. 15 or 16 in the Sequence Table, for example, based on the sequences of N-terminal and C-terminal of DNA sequence of Ribosomal Protein L7/L12 of *Neisseria* *gonorrhoeae* in the same manner as in the case of *Haemophilus influenzae*, and *Streptococcus pneumoniae*, and processing thereafter in exactly the same manner as in the case of *Haemophilus influenzae* or *Streptococcus pneumoniae.*

Particularly, the gene sequence of ribosomal protein L7/L12 of *Neisseria meningitides*, which belongs to the same *Neisseria* genus as *Neisseria gonorrhoeae* is disclosed and readily available on the Internet. The entire base sequence and the corresponding amino acid sequence of the ribosomal protein L7/L12 gene of *Neisseria meningitidis* are shown in "Sequences No. 9 and No. 10." Here, comparing the entire base sequence for ribosomal protein L7/L12 genes of *Neisseria meningitidis* and *Neisseria gonorrhoeae*, only difference in the amino acid sequence is that Neisseria gonorrhoeae has alanine for the 115th amino acid from the N-terminal, whereas *Neisseria meningitidis* has glutamic acid. Therefore, it can be concluded that the antibody for the ribosomal protein L7/L12 of *Neisseria gonorrhoeae* which can specifically detects *Neisseria gonorrhoeae* is the antibody which identifies alanine at 115 from the N-terminal and the amino acid region including the alanine of ribosomal protein L7/L12 as an epitope.

Antibody made based on the present invention can be used in all known types of immunoassay, such as aggregation whereby said antibody is adsorbed on polystyrene latex, ELISA, which is a conventional technology performed in a microtiter plate, conventional immunochromatography, sandwich assay, whereby said antibody labeled with colored particles or particles that have coloring capability, or enzymes or phosphor, and magnetic particles coated with capture antibody are used, etc.

Moreover, antibody that is made based on the present invention can simultaneously function in any of these immunoassay methods as a so-called capture antibody that captures said antigen protein in solid or liquid phase and a so-called enzyme-labeled antibody by modification using an enzyme, such as peroxidase and alkali phosphatase, etc., by conventional methods.

### BEST MODE FOR CARRYING OUT THE INVENTION

The following examples are given to explain the present invention in actual terms, the present invention not being restricted to these examples.

### Example 1

### Cloning of ribosomal protein L7/L12 genes from Haemophilus influenzae

After inoculating an appropriate amount of *Haemophilus influenzae* strain ATCC9334 (IID984) (obtained from Tokyo University School of Medicine Laboratories) in a chocolate agar culture medium, the strain was cultivated for 24 hours in a CO₂ incubator under conditions of 37 °C and 5.0 % CO₂. The colonies that grew were suspended in a TE buffer (manufactured by Wako Pure Chemical Co., Ltd.) to a final concentration of approximately 5 x 10⁹ CFU/ml. Approximately 1.5 ml of this suspension was transferred to a microcentrifuge tube and centrirfuged for 2 minutes at 10,000 rpm. The supernatant was discarded. The sediment was resuspended in 567 µl TE buffer. Then 30 µl 10 % sodium dodecylsulfate (SDS) and 3 µl 20 mg/ml Proteinase K solution were added and thoroughly mixed. The suspension was incubated for another hour at 37 °C. Next, after adding 80 µl 10 % cetyl trimethyl ammonium bromide/0.7 M NaCl solution and thoroughly mixing the product, it was incubated for 10 minutes at 65 °C. Next, 700 µl chloroform-isoamyl alcohol solution at a volume ratio of 24:1 was added and stirred well. The solution was centrifuged for 5 minutes (while being kept at 4 °C) at 12,000 rpm using a microcentrifugation device and the aqueous fraction was transferred to a new microtube. Isopropanol was added to the fraction at 0.6-times its volume and the tube was vigorously shaken to form sediment of the DNA. The white DNA sediment was scooped with a glass rod and transferred to a different microcentrifuge tube containing 1 ml 70 % ethanol (cooled to -20 °C).

Next, the product was centrifuged for 5 minutes at 10,000 rpm and the supernatant was gently removed. Then another 1 ml 70 % ethanol was added and the product was centrifuged for 5 more minutes.

Once the supernatant had been removed, the sediment was dissolved in 100 µl TE buffer to obtain the DNA solution. The concentration of the genomic DNA solution was determined quantitatively according to E5, Spectrophotometric determination of the amount of DNA or RNA "Molecular cloning: A laboratory manual," 1989, Eds. Sambrook, J., Fritsch, E.F., and Maniatis, T., Cold Spring Harbor Laboratory Press.

PCR (polymerase chain reaction) was performed using 10 ng of this genomic DNA. Taq polymerase (Takara Co., ltd., code R001A) was employed for PCR. Then 5 µl of buffer, 4 µl dNTP mixture, and 200 pmol each of synthetic oligonucleotide-A, shown in Sequence No. 11 of the Sequence Table and synthetic oligonucleotide-B, shown in Sequence No. 12 of the Sequence table were added to the enzyme to bring the final volume to 50 µl.

This mixture was cycled 5 times with a TaKaRa PCR Thermal Cycler 480 for 1 minute at 95 °C, 2 minutes at 50 °C, and 3 minutes at 72 °C and was then cycled 25 times for 1 minute at 95 °C, 2 minutes at 60 °C, and 3 minutes at 72 °C. Electrophoresis was performed in 1.5 % agarose gel using some of this PCR product. This product was then stained with ethidium bromide (Nihon Gene Co., ltd.) and observed under ultraviolet rays to confirm amplification of approximately 400 bp cDNA. After digestion treatment with restriction endonucleases BamHI and XhoI, electrophoresis was performed in 1.5 % agarose gel and staining with ethidium bromide was carried out. An approximately 370 bp band was cut out from the gel. This band was purified with Suprec01 (Takara Co., Ltd.) and then inserted into pGEX-4T-1 (Pharmacia), which is a commercial vector. This same vector can function as an expression vector for the desired molecule, which can express fused protein with GST protein, by insertion of the desired gene fragment into the appropriate restriction endonuclease site.

Actually, vector pGEX-4T-1 and the previous DNA were mixed together at a molar ratio of 1:3 and DNA was inserted into the vector with T4 DNA ligase (Invitrogen Co.). Vector pGEX-4T-1 into which DNA had been inserted was genetically introduced to *Escherichia coli* one-shot competent cells (Invitrogen Co., Ltd.) and then inoculated in a plate of L-Broth (Takara Co., Ltd.) semi-sold culture plate containing 50 µg/ml ampicillin (Sigma). The plate was then set-aside at 37 °C for 12 hours and the colonies that grew were selected at random and inoculated into 2 ml L-Broth liquid culture medium containing the same concentration of ampicillin. Shake cultivation was performed at 37 °C for 8 hours and the bacteria were recovered and the plasmid was separated using Wizard Miniprep (Promega) in accordance with the attached literature. The plasmid was cleaved with restriction endonuclease BamHI/XhoI. Insertion of said PCR product was confirmed by cutting out approximately 370 bp DNA. The base sequence of the DNA that had been inserted was determined using said clone.

Determination of the base sequence of the inserted DNA fragment was performed using the Fluorescence Sequencer of Applied Biosystems. The sequence sample was prepared using PRISM, Ready Reaction Dye Terminator Cycle Sequencing Kit (Applied Biosystems). First, 9.5 µl reaction stock solution, 4.0 µl T7 promoter primer at 0.8 pmol/µl (Gibco BRL) and 6.5 µl of template DNA for sequencing at 0.16 g/µl were added to a microtube with a capacity of 0.5 ml, mixed and superposed with 100 µl mineral oil. PCR amplification was performed for 25 cycles, where one cycle consisted of 30 seconds at 96 °C, 15 seconds at 55 °C, and 4 minutes at 60 °C. The product was then kept at 4 °C for 5 minutes. After the reaction was completed, 80 µl sterilized pure water was added and stirred. The product was centrifuged and the aqueous layer was extracted 3 times with phenol-chloroform. 10µl <microliters> 3 M sodium acetate (pH 5.2) and 300 µl ethanol were added to 100 µl aqueous layer and stirred. The product was then centrifuged for 15 minutes at room temperature and 14,000 rpm and the sediment was recovered. Once the sediment was washed with 75 % ethanol, it was dried under a vacuum for 2 minutes to obtain the sequencing sample. The sequencing sample was dissolved in formamide containing 4 µl 10 mM EDTA and denatured for 2 minutes at 90 °C. This was then cooled in ice and applied to sequencing.

One of the 5 clones obtained had homology of the sequence with the probe used for PCR. In addition, DNA sequences extremely similar to the gene sequence of ribosomal protein L7/L12 gene of the other microorganisms, for example, *Neisseria gonorrhoeae*, were discovered. The entire base sequence and the corresponding amino acid sequence of the structural gene moiety are as shown in Sequence ID No. 17 and No. 18 of the Sequence Table. This gene fragment clearly codes for *Haemophilus influenzae* ribosomal protein L7/L12.

### Example 2

### Mass expression in Escherichia coli and purification of ribosomal protein L7/L12 from Haemophilus influenzae

Fifty milliliters *Escherichia coli* into which expression vector had been inserted were cultivated overnight in LB at 37 °C. Then 500 ml YT medium at concentration that was twice that of the aforementioned culture was heated at 37 °C for 1 hour. Fifty milliliters of the *Escherichia coli* solution that had been cultivated overnight were introduced to 500 ml of the aforementioned medium. One hour later, 550 µl 100 mM isopropyl β-(D)-thiogalactopyranoside (IPTG) were introduced and cultivated for 4 hours. The product was then recovered and introduced to centrifugation tube at each 250 ml and centrifuged for 10 minutes at 7,000 rpm.

The supernatant was discarded and dissolved in 25 ml each 50 mM Tris-HCl at a pH of 7.4 and Lysis buffer containing 25 % sucrose.

Furthermore, 1.25 ml 10 % Nonidet P-40 (NP-40) and 125 µl 1 M MgCl₂ were added and transferred to a plastic tube. Sonication was performed 1 minute x 5 times while ice cold. The product was centrifuged for 15 minutes at 12,000 rpm and the supernatant was recovered.

Next, the aforementioned supernatant was adsorbed on a glutathione agarose column conditioned with phosphate-buffered saline (PBS).

Then the column was washed with twice the bed volume using a washing solution containing 20 mM Tris buffer at a pH of 7.4, 4.2 mM MgCl₂, and 1 mM dithiothreithol (DTT). Elution was performed with 50 mM Tris buffer at a pH of 9.6 containing 5 mM glutathione. The protein content in the fraction was determined by the pigment bonding method (Bradford method; BioRad Co.) and the main fraction was acquired.

Purity of the purified ribosomal protein L7/L12/GST fused protein that was obtained was confirmed by electrophoresis to be approximately 75 %, showing that a purity satisfactory for an immunogen could be guaranteed.

### Example 3

### Preparation of monoclonal antibody to ribosomal protein L7/L12 of Haemophilus influenzae

First, 100 µg fused protein antigen of ribosomal protein L7/L12/GST of *Haemophilus influenzae* where dissolved in 200 µl PBS and then 200 µl Freund's complete adjuvant were added and mixed and emulsification was performed. Two-hundred microliters were injected intraperitoneally to immunize mice.

Then the same emulsion antigen was intraperitoneally injected after 2 weeks, after 4 weeks, and after 6 weeks. Two-fold the concentration of antigen emulsion was injected intraperitoneally after 10 weeks and after 14 weeks. The spleen was excised 3 days after the final immunization and submitted to cell fusion.

After thoroughly mixing 2 x 10⁷ myeloma cells per 10⁸ spleen cells from mice, which had been recovered aseptically, in a glass tube, the mixture was centrifuged for 5 minutes at 1,500 rpm and the supernatant was discarded. The cells were thoroughly mixed.

The myeloma cells used for cell fusion were obtained by cultivation of cell strain NS-1 with an RPMI 1640 culture medium containing 10 % FCS, cultivating this product beginning 2 weeks before cell fusion using an RPMI 1640 medium containing 0.13 mM azaguanine, 0.5 µg/ml MC-210, and 10 % FCS for 1 weeks, and then further cultivating the cell strain for 1 week with an RPMI 1640 medium containing 10 % FCS.

Thirty milliliters of RPMI 1640 culture medium 50 ml that had been kept at 37 °C were added to the mixed cell sample and centrifuged at 1,500 rpm. After removal of the supernatant, 1 ml 50 % polyethylene glycol that had been kept at 37 °C was added and stirred for 2 minute. 10 ml RPMI 1.640 medium kept at 37 °C were added and the solution was vigorously mixed for approximately 5 minutes as it was suctioned and evacuated from a sterile pipette.

After centrifugation for 5 minutes at 1,000 rpm and removal of the supernatant, 30 ml HAT medium were added to bring the cell concentration to 5 x 10⁶ cells/ml. This mixture was stirred till uniform and then poured, 0.1 ml at a time, into a 96-well culture plate and cultivated at 37 °C under 7 % CO₂. HAT medium was added, 0.1 ml at a time, on Day 1 and at Week 1 and Week 2.

Then the cells that had produced the desired antibody were screened by ELISA.

Solutions of GST fusion ribosomal protein L7/L12 and GST protein of *Haemophilus influenzae* dissolved in PBS containing 0.05 % sodium azide diluted to 10 µg/ml were poured, each 100 µl at a time, into separate 96-well plates and adsorbed overnight at 4 °C.

After removal of the supernatant, 200 µl of 1 % bovine serum albumin solution (in PBS) were added and reacted and blocked for 1 hour at room temperature. After removal of the supernatant, the product was washed with washing, solution (0.02 % Tween 20, PBS). One-hundred microliters culture solution of fused cells were added to this and reacted for 2 hours at room temperature. The supernatant was removed and washed with washing solution. Next, 100 µl peroxidase-labeled goat anti-mouse IgG antibody solution at a concentration of 50 ng/ml were added and the solution was reacted for 1 hour at room temperature. The supernatant was removed and the product was again washed with washing solution. Then TMB solution (KPL Co., ltd) was added, 100 µl at a time, and the mixture was reacted for 20 minutes at room temperature. After coloration, 100 µl 1 N sulfuric acid were added to stop the reaction and absorbance at 450 nm was determined.

As a result, positive wells that only reacted with GST fusion ribosomal protein L7/L12 and did not react with GST protein were detected, and it was concluded that antibody to ribosomal protein L7/L12 is present.

Therefore, the cells in the positive wells were recovered and cultivated with HAT medium in a 24-well plastic plate. The fused medium that had been cultivated was diluted with HT medium to a cell count of approximately 20 cells/ml and then mixed with 10⁶ six-week-old mouse thyroid cells suspended in HT Cultivation medium in a 96-well culture plate. Culture was performed for 2 weeks at 37 °C under conditions of 7 % CO₂.

Antibody activity in the culture supernatant was similarly determined by the aforementioned ELISA method and the cells that showed positive reaction with ribosomal protein L7/L12 were recovered.

Furthermore, the same dilution test and cloning procedure was repeated to obtain a total of 5 clones of hybridoma HIRB-1 - 5.

### Example 4

### Reaction of monoclonal antibody that reacts with ribosomal protein L7/L12 of Haemophilus influenzae, with Neisseria gonorrhoeae and other microorganisms

Monoclonal antibody was. produced and recovered in accordance with standard methods using the positive hybridoma cells obtained as previously described.

Basically, 5 x 10⁶ cells that had been subcultured using RPMI 1640 culture medium (containing 10 % FCS) were intraperitoneally injected into Balb/C mice that had been intraperitoneally injected with 0.5 ml Pristane 2 weeks earlier. Ascites was recovered 3 weeks later and the centrifugation supernatant was obtained.

The solution containing antibody that was obtained was adsorbed in a Protein A column (5 ml bed, Pharmacia) and washed with 3-bed volume of PBS. Then eluted with citrate buffer at pH 3, the antibody fraction was recovered and the monoclonal antibody produced by each hybridoma was obtained.

The monoclonal antibody derived from these 5 strains of hybridoma was used in ELISA.

The sandwich assay method was used to assess the monoclonal antibody. The monoclonal antibody that was prepared was used as an enzyme-labeled antibody by being chemically bound to peroxidase.

That is, enzyme labeling was performed in accordance with the method in "Analytical Biochemistry" 132 (1983), 68-73 with the reagent S-acetylthioacetic acid N-hydroxysuccinimide for binding using horseradish peroxidase (Sigma Grade VI). By means of the ELISA reaction, a solution of commercial anti-*Haemophilus influenzae* polyclonal antibody which dissolved in PBS containing 0.05 % sodium azide, (Biodesign, rabbit) was diluted to a concentration of 10 µg/ml and poured,100 µl at a time, into a separate 96-well plate and adsorbed overnight at 4 °C.

After removal of the supernatant, 200 µl 1 % FCS solution (in PBS) were added and reacted and blocked for 1 hour at room temperature. The supernatant was removed and the product was washed with washing solution (0.02 % Tween 20, PBS). One-hundred microliters of antigen solution, which had been obtained by adding Triton X-100 to culture solutions of each species of microorganism to a concentration of 0.3 % and then extracting the solution for 5 minutes at room temperature, were added to this and reacted for 2 hours at room temperature. The supernatant was removed and the product was further washed with washing solution. Then 100 µl peroxidase-labeled anti-ribosomal protein L7/L12 antibody solution at 5 µg/ml were added and reacted for 1 hour at room temperature. The supernatant was removed and the product was washed with washing solution. TMB (KPL Co.) solution was added, 100 µl at a time, and reacted for 20 minutes at room temperature. After coloration, 100 µl 1 N sulfuric acid were added to stop the reaction. Absorbance at 450 nm was determined.

As a result, as shown in Table 1 it is clear that when monoclonal antibody derived from hybridoma HIRB-2 was used as the enzyme-labeled antibody, all strains of *Haemophilus influenzae* tested were detected at a sensitivity of 10⁶ bacteria/ml, while reactivity of other bacteria belonging to the genus Neisseria and other microorganisms could not be detected, even at high concentrations of 10⁸ bacteria/ml and therefore, antibody with specific reactivity to *Haemophilus influenzae* can be obtained by using monoclonal antibody to ribosomal protein L7/L12.

**Table 1**

| Results of Detection (10⁶ cells/ml) | | |
|---|---|---|
| *Haemophilus influenzae* | ATCC9327 | + |
| *Haemophilus influenzae* | ATCC9334 | + |
| *Haemophilus influenzae* | ATCC9007 | + |
| *Haemophilus influenzae* | ATCC9332 | + |
| *Haemophilus influenzae* | ATCC8142 | + |
| *Haemophilus influenzae* | ATCC9833 | + |

| Results of Detection (10⁸ cells/ml) | | |
|---|---|---|
| *Neisseria meningitides* | ATCC13090 | - |
| *Neisseria lactamica* | ATCC30011 | - |
| *Neisseria mucosa* | ATCC35611 | - |
| *Neisseria sicca* | ATCC9913 | - |
| | | |
| *Branhamella catarrharis* | ATCC25240 | - |
| *Neisseria gonorrhoeae* | ATCC9793 | - |
| *Escherichia coli* | ATCC25922 | - |
| *Klebsiella pneumoniae* | ATCC13883 | - |
| (+: Positive; -: Negative) | | |

### Example 5

### Cloning of ribosomal protein L7/L12 genes from Streptococcus pneumponiae, mass expression in Escherichia coli and purification of the same protein and preparation of monoclonal antibody to the same protein

After inoculating an appropriate amount of *Streptococcus pneumoniae* strain IID555 (obtained from Tokyo University School of Medicine Laboratories) in a blood agar culture medium, the strain was cultivated for 48 hours in an incubator at 37 °C. The colonies that grew were suspended in a TE buffer to a final concentration of approximately 5 x 10⁹ CFU/ml. Approximately 1.5 ml of this suspension was transferred to a microcentrifuge tube and centrifuged for 2 minutes at 10,000 rpm. The supernatant was discarded. The sediment was resuspended in 567 µl TE buffer. Then 30 µl 10 % SDS and 3 µl 20 mg/ml Proteinase K solution were added and thoroughly mixed. The suspension was incubated for another hour at 37 °C. Next, after adding 80 µl 10 % cetyl trimethyl ammonium bromide/0.7 M NaCl solution and thoroughly mixing the product, it was incubated for 10 minutes at 65 °C. Next, 700 µl chloroform-isoamyl alcohol solution at a volume ratio of 24:1 was added and stirred well. The solution was centrifuged for 5 minutes (while being kept at 4 °C) at 12,000 rpm using a microcentrifugation device and the aqueous fraction was transferred to a new microtube. Isopropanol was added to the fraction at 0.6-times its volume and the tube was vigorously shaken to form sediment of the DNA. The white DNA sediment was scooped with a glass rod and transferred to a different microcentrifuge tube containing 1 ml 70 % ethanol (cooled to -20 °C).

Next, the product was centrifuged for 5 minutes at 10,000 rpm and the supernatant was gently removed. Then another 1 ml 70 % ethanol was added and the product was centrifuged for 5 more minutes. Once the supernatant had been removed, the sediment was dissolved in 100 µl TE buffer to obtain the DNA solution. The concentration of the genomic DNA solution was determined quantitatively in accordance with E5, Spectrophotometric determination of the amount of DNA or RNA, "Molecular cloning: A laboratory manual," 1989, Eds. Sambrook, J., Fritsch, E.F., and Maniatis, T., Cold Spring Harbor Laboratory Press.

PCR was performed using 10 ng of this genomic DNA. Taq polymerase (Takara Co., ltd., code R001A) was employed for PCR. Then 5 µl of buffer attached to enzyme, 4 µl dNTP mixture attached to enzyme, and 200 pmol each of synthetic oligonucleotide C shown in Sequence No. 13 of the Sequence Table and synthetic oligonucleotide D shown in Sequence No. 14 of the Sequence table were added to the enzyme to bring the final volume to 50 µl.

This mixture was cycled 5 times with a TaKaRa PCR Thermal Cycler 480 for 1 minute at 95 °C, 2 minutes at 50 °C, and 3 minutes at 72 °C and was then cycled 25 times for 1 minute at 95 °C, 2 minutes at 60 °C, and 3 minutes at 72 °C, Electrophoresis was performed in 1.5 % agarose gel using some of this PCR product. This product was then stained with ethidium bromide (Nihon Gene Co., ltd.) and observed under ultraviolet rays to confirm amplification of approximately 400 bp cDNA. After digestion treatment with restriction endonucleases BamHI and XhoI, electrophoresis was performed in 1.5 % agarose gel and staining with ethidium bromide was carried out. An approximately 370 bp band was cut out from the gel. This band was purified with Suprec01 (Takara Co., Ltd.) and then inserted into pGEX-6P-1 (Pharmacia), which is a commercial vector.

This same vector can function as an expression vector for the desired molecule, which can express fused protein with GST protein, by insertion of the desired gene fragment into the appropriate restriction endonuclease site. Actually, vector pGEX-6P-1 and the previous DNA were mixed together at a molar ratio of 1:5 and DNA was inserted into the vector with T4 DNA ligase (Invitrogen Co.). Vector pGEX-4T-1 into which DNA had been inserted was genetically introduced to *Escherichia coli* One-Shot Competent Cells (Invitrogen Co., Ltd.) and then inoculated in a plate of L-Broth (Takara Co., Ltd.) semi-sold culture plate containing 50 µg/ml ampicillin (Sigma). The plate was then set aside at 37°C for 12 hours and the colonies that grew were selected at random and inoculated into 2 ml L-Broth liquid culture medium containing the same concentration of ampicillin. Shake cultivation was performed at 37°C for 8 hours and the bacteria was recovered and the plasmid was separated using Wizard Miniprep (Promega Co.,) in accordance with the attached literature. The plasmid was cleaved with restriction endonuclease BamHI/XhoI. Insertion of said PCR product was confirmed by cutting out approximately 370 bp DNA. The base sequence of the DNA that had been inserted was determined using said clone.

Determination of the base sequence of the inserted DNA fragment was performed using the Fluorescence Sequencer of Applied Biosystems. The sequence sample was prepared using PRISM, Ready Reaction Dye Terminator Cycle Sequencing Kit (Applied Biosystems). First, 9.5 µl reaction stock solution, 4.0 µl T7 promoter primer at 0.8 pmol/µl (Gibco BRL) and 6.5 µl of template DNA for sequencing at 0.16 µg/µl were added to a microtube with a capacity of 0.5 ml, mixed and superposed with 100 µl mineral oil. PCR amplification was performed for 25 cycles, where one cycle consisted of 30 seconds at 96 °C, 15 seconds at 55 °C, and 4 minutes at 60 °C. The product was then kept at 4 °C for 5 minutes. After the reaction was completed, 80 µl sterilized pure water was added and stirred. The product was centrifuged and the aqueous layer was extracted 3 times with phenol-chloroform. Ten microliters 3 M sodium acetate (pH 5.2) and 300 µl ethanol were added to 100 µl aqueous layer and stirred. The product was then centrifuged for 15 minutes at room temperature and 14,000 rpm and the sediment was recovered, Once the sediment was washed with 75 % ethanol, it was dried under a vacuum for 2 minutes to obtain the sequencing sample. The sequencing sample was dissolved in formamide containing 4 µl 10 mM EDTA and denatured for 2 minutes at 90 °C. This was then cooled in ice and submitted to sequencing.

One of the 7 clones obtained had homology of the sequence with the probe used for PCR. In addition, DNA sequences extremely similar to the gene sequence of ribosomal protein L7/L12 gene of the other microorganisms, for example, *Neisseria gonorrhoeae*, were discovered. The entire base sequence and the corresponding amino acid sequence of the structural gene moiety are as shown in Sequence ID No. 19 and No. 20 of the Sequence Table. This gene fragment clearly codes for *Streptococcus pneumoniae* ribosomal protein L7/L12.

50 ml *Escherichia coli* into which expression vector had been inserted was cultivated overnight in a two-fold concentration YT medium at 37 °C. Then, 450 ml of the two-fold concentration YT medium was heated at 37 °C for 1 hour. 50 ml of the *Escherichia coli* solution that had been cultivated overnight was introduced to 450 ml of the aforementioned medium. After cultivation for one hour at 37 °C, 100 µl 500 mM IPTG was introduced and cultivated for 4 hours at 25 °C. The product was then recovered and introduced to centrifugation tube at each 250 ml and centrifuged for 20 minutes at 5000 rpm. The supernatant was discarded and dissolved in 25 ml each 50 mM Tris-HCl at a pH of 7.4 and Lysis buffer containing 25 % sucrose.

Furthermore, 1.25 ml 10 % NP-40 and 125 µl 1 M MgCl₂ were added and transferred to a plastic tube. Sonication was performed 1 minute x 5 times while ice cold. The product was centrifuged for 15 minutes at 12,000 rpm and the supernatant was recovered.

Next, the aforementioned supernatant was adsorbed on a glutathione sepharose column (manufactured by Pharmacia) conditioned with PBS. Then, the column was washed with PBS three times the bed volume. Elution was performed with 50 mM Tris-HCl at a pH of 8.0 containing 10 mM glutathione. The protein content in the fraction was determined by the pigment bonding method (Bradford method; BioRad Co.) and the main fraction was acquired. The main fraction was dialyzed three times against 3 L PBS.

1 ml of a cleavage buffer containing 500 mM Tris-HCl (pH 7.0), 1.5 M NaCl, 10 mM EDTA, and 10 mM DTT was added to 10 ml of 1 mg/ml solution of the resulting GST fusion ribosomal protein L7/L12. 100 µl of 2 u/µl PreScission Protease (manufactured by Pharmacia company) was further added and reacted at 4 °C to separate the GST moiety from ribosomal protein L7/L12.

The reaction solution was passed through a glutathione sepharose column which had been conditioned with PBS. The solution coming out from the column was recovered. One-bed volume of PBS was passed through and also recovered. Purity of the purified ribosomal protein L7/L12 that was obtained was confirmed by electrophoresis to be approximately 90 %, showing that a purity satisfactory for an immunogen could be guaranteed.

First, 100 µg protein antigen of ribosomal protein L7/L12 of *Streptococcus pneumoniae* was dissolved in 200 µl PBS and then 200 µl Freund's complete adjuvant was added and mixed and emulsification was performed. 200 µl was intraperitoneally injected to immunize mice. Then, the same emulsion antigen was intraperitoneally injected after 2 weeks, after 4 weeks, and after 6 weeks. A two-fold concentration antigen emulsion was further injected intraperitoneally after 10 weeks and after 14 weeks. The spleen was excised 3 days after the final immunization and submitted to cell fusion.

After thoroughly mixing 2 x 10⁷ myeloma cells per 10⁸ spleen cells from mice, which had been recovered aseptically, in a glass tube, the mixture was centrifuged for 5 minutes at 1500 rpm and the supernatant was discarded. The cells were thoroughly mixed.

The myeloma cells used for cell fusion were obtained by cultivation of cell strain NS-1 with an RPMI 1640 culture medium containing 10 % FCS, cultivating this product beginning 2 weeks before cell fusion using an RPMI 1640 medium containing 0.13 mM azaguanine, 0.5 µg/ml MC-210, and 10 % FCS for 1 weeks, and then further cultivating the cell strain for 1 week with an RPMI 1640 medium containing 10 % FCS. 30 ml of RPMI 1640 culture medium 50 ml that had been kept at 37 °C was added to the mixed cell sample and centrifuged at 1,500 rpm. After removal of the supernatant, 1 ml 50 % polyethylene glycol that had been kept at 37 °C was added and stirred for 2 minute. 10 ml RPMI 1640 medium kept at 37 °C was added and the solution was vigorously mixed for approximately 5 minutes as it was suctioned and evacuated using a sterile pipette.

After centrifugation for 5 minutes at 1,000 rpm and removal of the supernatant, 30 ml HAT medium were added to bring the cell concentration to 5 x 10⁶ cells/ml. This mixture was stirred till uniform and then poured, 0,1ml at a time, into a 96-well culture plate and cultivated at 37 °C under 7 % CO₂. HAT medium was added, 0.1 ml at a time, on Day 1 and at Week 1 and Week 2.

Then, the cells that had produced the desired antibody were screened by ELISA. Solutions of ribosomal protein L7/L12 of *Streptococcus pneumoniae* dissolved in PBS containing 0.05 % sodium azide diluted to 10 µg/ml were poured, 100 µl at a time, into separate 96-well plates and adsorbed overnight at 4 °C. After removal of the supernatant, 200 µl 1 % bovine serum albumin solution (in PBS) were added and reacted and blocked for 1 hour at room temperature. The supernatant was removed and the product was washed with a washing solution (0.02 % Tween 20, PBS). 100 µl of a culture solution of fusion cells was added and reacted for two hours at room temperature. The supernatant was removed and the product was further washed with a washing solution. Then, 100 µl of a peroxidase-labeled goat anti-mouse antibody solution at 50 ng/ml was added and reacted for one hour at room temperature. The supernatant was removed and the product was washed with a washing solution. TMB (KPL) solution was added, 100 µl at a time, and reacted for 20 minutes at room temperature. After coloration, 100 µl 1 N sulfuric acid were added to stop the reaction. Absorbance at 450 nm was determined.

As a result, positive wells that reacted with ribosomal protein L7/L12 were detected, confirming presence of the antibody to ribosomal protein L7/L12.

Therefore, the cells in the positive wells were recovered and cultivated with HAT medium in a 24-well plastic plate. The fused medium that had been cultivated was diluted with an HT medium to a cell count of approximately 20 cells/ml and then mixed with 10⁶ six-week-old mouse thyroid cells suspended in the HT medium in a 96-well culture plate. The cells were cultivated for 2 weeks at 37 °C under the conditions of 7 % CO₂. The antibody activity in the culture supernatant was determined by the aforementioned ELISA method and the cells that showed a positive reaction with ribosomal protein L7/L12 were recovered.

Furthermore, the same dilution and cloning procedure was repeated to obtain a total of 4 clones of hybridoma AMSP-1 to 4.

### Example 6

### Reaction of monoclonal antibody that reacts with ribosomal protein L7/L12 of Streptococcus pneumoniae, with Streptococcus pneumoniae and other microorganisms

A monoclonal antibody was produced and recovered in accordance with standard methods using the positive hybridoma cells obtained as previously described.

Specifically, cells subcultured in RPMI 1640 medium (containing 10 % FCS) was diluted with a serum-free medium to about 2 x 10⁵ cells/ml, 3.3 x 10⁵ cells/ml, and 5 x 10⁵ cells/ml in 25 cm² culture flasks, and the total volume was made 5 ml. After cells were grown for 3 to 5 days in 7 % CO₂ at 37 °C, a flask which contains the least number of original cells was selected among flasks in which cells were grown. The same procedure was repeated until the cells diluted to 2 x 10⁵ cells/ml grow to 2 x 10⁶ cells/ml in 3 to 4 days, thereby adapting the cells with the serum-free medium. Next, cloning was performed in a 96-well plate for bacteria cultivation to select cells exhibiting fastest growth and a highest antibody titer. The selected cells were grown in a 24-well plate and diluted with a serum-free medium in a 25 cm² culture flask to a concentration of about 2 x 10⁵ cells/ml and the total volume was made 10 ml. After incubation for 3 to 4 days in 7 % CO₂ at 37 °C to a concentration of 1 x 10⁶ cells/ml, the culture broth 100 ml, 1 x 10⁶ cells/ml was transferred to a bottle for mass cultivation which were grown in the same manner in a 75 cm² flask. 100 ml of a serum-free medium was added to the mixture, which was incubated at 37 °C for two days while stirring. 200 ml of the serum-free medium was added again and the mixture was incubated for a further two days. The culture broth was divided into four aliquot, the serum-free medium was added to each portion, followed by incubation for two days. After further addition of 400 ml of the serum-free medium, the culture broth was incubated for 6 days. The culture broth was collected and centrifuged at 10,000 rpm for 15 minutes to obtain a culture supernatant that contain the target antibody. After the addition of sodium azide to final concentration 0.1 %, the culture supernatant was stored at 4 °C. 100 ml of the solution containing the antibody that was obtained was 5-fold diluted with PBS and adsorbed in a Protein G column (5 ml bed, Pharmacia) and washed with 3-bed volume of PBS. Then eluted with citrate buffer at a pH 3, the antibody fraction was recovered, and monoclonal antibody produced by each hybridoma was obtained. The monoclonal antibodies originating from the four hybridoma clones were evaluated according to the OIA method described in Publication of International Patent Application Japanese Laid-open (Toku-Hyou) No. 509565/1995.

Specifically, the OIA method comprises preparing a reactive substrate by reacting an antibody for capture on a silicon wafer having a thin film layer of silicon nitride, causing this substrate to react with an antigen which is an extract of microorganisms for a prescribed period of time, causing the captured antigen to react with an antibody (an amplification reagent) which is an enzyme-labeled antibody, and finally adding a substrate solution to produce a thin-film precipitate. The antigen-antibody reaction can be judged visually by a degree of light interference color produced in the precipitate.

The monoclonal antibody preparation was used and evaluated as a capture antibody to be immobilized on a silicon wafer having a silicon nitride thin film layer in the OIA method. Moreover, peroxidase-labeled AMGC-1 monoclonal antibody which can non-specifically react with ribosomal proteins L7/L12 protein of a variety of microorganisms described in Reference Example was used as the detect antibody. That is, enzyme labeling was performed in accordance with the method in "Analytical Biochemistry" 132 (1983), 68-73 with the reagent S-acetylthioacetic acid N-hydroxysuccinimide for binding using horseradish peroxidase (Sigma Grade VI).

In the OIA reaction, monoclonal antibody in a PBS containing 0.05 % sodium azide was diluted with 0.1 M HEPES (pH 8.0) to a concentration of 10 µg/ml and added onto a silicone wafer which has a thin film layer of silicon nitride, 50 µl at a time, to react for 30 minutes at room temperature, followed by washing with distilled water and use.

15 µl of antigen solution, which had been obtained by adding 0.5% Triton X-100 to culture solutions of various species of microorganisms and then extracting the solution for 5 minutes at room temperature, was added to the specimen obtained in the above-described procedure and reacted for 10 minutes at room temperature. Then, 15 µl of 20 µg/ml peroxidase-labeled AMGC1 anti-body was added and reacted for 10 minutes. After washing with distilled water, a substrate solution (KPL) was added, 15 µl at a time, and reacted for 5 minutes at room temperature. The product was washed with distilled water to judge the concentration of detection signals as an intensity of light interference by naked eyes.

As a result, as shown in Table 2 it is clear that when monoclonal antibody derived from hybridoma AMSP-2 was used as the capture antibody, all strains of *Streptococcus pneumoniae* tested were detected at a sensitivity of 10⁶ bacteria/ml, while reactivity of other bacteria could not be detected at a higher concentration of 10⁸ bacteria/ml. Thus, the antibody with specific reactivity to *Streptococcus pneumoniae* was confirmed to have been obtained by using the monoclonal antibody to ribosomal protein L7/L12.

The hybridoma AMSP-2 which produces the monoclonal antibody specific to *Streptococcus pneumoniae* has been deposited with National Institute of Bioscience and Human-Technology, the Agency of Industrial Science and Technology, the Ministry of International Trade and Industry, Japan, on July 28, 1999, with the disposition number FERM BP-6807.

**Table 2**

| Results of Detection (10⁶ cells/ml) | | |
|---|---|---|
| *Streptococcus pneumoniae* | ATCC27336 | + |
| *Streptococcus pneumoniae* | IID554 | + |
| *Streptococcus pneumoniae* | IID555 | + |
| *Streptococcus pneumoniae* | IID556 | + |
| *Streptococcus pneumoniae* | IID557 | + |
| *Streptococcus pneumoniae* | IID558 | + |
| *Streptococcus pneumoniae* | IID559 | + |
| *Streptococcus pneumoniae* | IID1603 | + |

| Results of Detection (10⁸ cells/ml) | | |
|---|---|---|
| *Escherichia coli* | ATCC25922 | - |
| *Enterococcus faecalis* | ATCC19433 | - |
| *Haemophilus influenzae* | ATCC10211 | - |
| *Klebsiella pneumoniae* | ATCC13883 | - |
| *Neisseria gonorrhoeae* | IID821 | - |
| *Neisseria lactamica* | ATCC23970 | - |
| *Neisseria meningitides* | ATCC13090 | - |
| *Pseudomonas aeruginosa* | ATCC27853 | - |
| *GroupB streptococcus* | ATCC12386 | - |
| *Staphylococcus aureus* | ATCC25923 | - |
| *Streptococcus pyogenes* | ATCC19615 | - |
| (+: Positive; -: Negative) | | |

### Example 7

### Cloning of ribosomal protein L7/L12 genes from Neisseria gonorrhoeae, mass expression in Escherichia coli and purification of the same protein and preparatian of monoclonal antibody to the same protein

After inoculating an appropriate amount of *Neisseria gonorrhoeae* strain IID821 (obtained from Tokyo University School of Medicine Laboratories) in a chocolate agar culture medium, the strain was cultivated for 24 hours in a CO₂ incubator under conditions of 37 °C and 5.0 % CO₂. The colonies that grew were suspended in a TE buffer to a final concentration of approximately 5 x 10⁹ CFU/ml. Approximately 1.5 ml of this suspension was transferred to a microcentrifugation tube and centrifuged for 2 minutes at 10,000 rpm. The supernatant was discarded. The sediment was resuspended in 567 µl TE buffer. Then 30 µl 10 % SDS and 3 µl 20 mg/ml Proteinase K solution were added and thoroughly mixed. The suspension was incubated for another hour at 37 °C.

Next, after adding 80 µl 10 % cetyl trimethyl ammonium bromide/0.7 M NaCl solution and thoroughly mixing the product, it was incubated for 10 minutes at 65 °C. Next, 700 µl chloroform-isoamyl alcohol solution at a volume ratio of 24:1 was added and stirred well. The solution was centrifuged for 5 minutes (while being kept at 4 °C) at 12,000 rpm using a microcentrifugation device and the aqueous fraction was transferred to a new microtube. Isopropanol was added to the fraction at 0.6-times its volume and the tube was vigorously shaken to form sediment of the DNA. The white DNA sediment was scooped with a glass rod and transferred to a different microcentrifugation tube containing 1 ml 70 % ethanol (cooled to -20 °C).

Next, the product was centrifuged for 5 minutes at 10,000 rpm and the supernatant was gently removed. Then another 1 ml 70 % ethanol was added and the product was centrifuged for 5 more minutes.

Once the supernatant had been removed, the sediment was dissolved in 100 µl TE buffer to obtain the DNA solution. The concentration of the genomic DNA solution was determined quantitatively in accordance with E5, Spectrophotometric determination of the amount of DNA or RNA, "Molecular cloning: A laboratory manual," 1989, Eds. Sambrook, J., Fritsch, E.F., and Maniatis, T., Cold Spring Harbor Laboratory Press.

PCR was performed using 10 ng of this genomic DNA. PCR was performed using Taq polymerase (Takara Co., Ltd., code R001A). Then, 5 µl of a buffer attached to enzyme, 4 µl of a dNTP mixture attached to enzyme, and 200 pmol each of synthetic oligonucleotide E shown in Sequence No. 15 of the Sequence Table and synthetic oligonucleotide F shown in Sequence No. 16 of the Sequence Table, which were designed based on the ribosomal protein L7/L12 DNA sequence of *Neisseria gonorrhoeae* acquired from Internet Information (Oklahoma University, *N. Gonorrhoeae* Genome Project, disclosed genomic DNA data) because of the similarity with ribosomal protein L7/L12 DNA sequence of other bacteria, were added to the enzyme to bring the final volume to 50 µl.

This mixture was cycled 5 times with a TaKaRa PCR Thermal Cycler 480 for 1 minute at 95 °C, 2 minutes at 50 °C, and 3 minutes at 72 °C and was then cycled 25 times for 1 minute at 95 °C, 2 minutes at 60 °C, and 3 minutes at 72 °C. Electrophoresis was performed in 1.5 % agarose gel using some of this PCR product. This product was then stained with ethidium bromide (Nihon Gene Co., ltd.) and observed under ultraviolet rays to confirm amplification of approximately 400 bp cDNA. After digestion treatment with restriction endonucleases BamHI and XhoI, electrophoresis was performed in 1.5 % agarose gel and staining with ethidium bromide was carried out. An approximately 370 bp band was cut out from the gel. This band was purified with Suprec01 (Takara Co., Ltd.) and then inserted into pGEX-4T-1 (Pharmacia), which is a commercial vector. Actually, vector pGEX-4T-1 and the previous DNA were mixed together at a molar ratio of 1:3 and DNA was inserted into the vector with T4 DNA ligase (Invitrogen Co.). Vector pGEX-4T-l into which DNA had been inserted was genetically introduced to *Escherichia coli* One-Shot Competent Cells (Invitrogen Co., Ltd.) and then inoculated in a plate of L-Broth (Takara Co., Ltd.) semi-sold culture plate containing 50 µg/ml ampicillin (Sigma). The plate was then set aside at 37 °C for 12 hours and the colonies that grew were selected at random and inoculated into 2ml L-Broth liquid culture medium containing the same concentration of ampicillin. Shake cultivation was performed at 37°C for 8 hours and the bacteria was recovered and the plasmid was separated using Wizard Miniprep in accordance with the attached literature. The plasmid was cleaved with restriction endonuclease BamHI/XhoI. Insertion of said PCR product was confirmed by cutting out approximately 370 bp DNA. The base sequence of the DNA that had been inserted was determined using said clone.

Determination of the base sequence of the inserted DNA fragment was performed using the Fluorescence Sequencer of Applied Biosystems. The sequence sample was prepared using PRISM, Ready Reaction Dye Terminator Cycle Sequencing Kit (Applied Biosystems). First, 9.5 µl reaction stock solution, 4.0 µl T7 promoter primer at 0.8 pmol/µl (Gibco BRL) and 6.5 µl template DNA for sequencing at 0.16 µg/µl were added to a microtube with a capacity of 0.5 ml and mixed. After layering with 100 µl mineral oil, PCR amplification was performed for 25 cycles, where one cycle consisted of 30 seconds at 96 °C, 15 seconds at 55 °C, and 4 minutes at 60 °C. The product was then kept at 4 °C for 5 minutes. After the reaction was completed, 80 µl sterilized pure water was added and stirred. The product was centrifuged and the aqueous layer was extracted 3 times with phenol-chloroform. Ten microliters 3 M sodium acetate (pH 5.2) and 300 µl ethanol were added to 100 µl aqueous layer and stirred. The product was then centrifuged for 15 minutes at room temperature and 14000 rpm and the sediment was recovered. Once the sediment was washed with 75 % ethanol, it was dried under a vacuum for 2 minutes to obtain the sequencing sample. The sequencing sample was dissolved in formamide containing 4 µl 10 mM EDTA and denatured for 2 minutes at 90 °C. This was then cooled in ice and submitted to sequencing. One of the 5 clones obtained had homology of the sequence with the probe used for PCR. In addition, DNA sequences extremely similar to the gene sequence of ribosomal protein L7/L12 gene of the other microorganisms, for example, *Haemophilus influenzae*, were discovered. The entire base sequence and the corresponding amino acid sequence of the structural gene moiety are as shown in Sequence No. 21 and No. 22 of the Sequence Table. This gene fragment clearly codes for ribosomal protein L7/L12 gene of *Neisseria gonorrhoeae.*

*Neisseria gonorrhoeae* fusion GST ribosome protein L7/L12 prepared by the same method as in Example 2 was obtained using the *Neisseria gonorrhoeae* fusion GST ribosomal protein L7/L12 expression vector constructed in this way.

Furthermore, hybridoma strain GCRB-3, which produces monoclonal antibody to ribosomal protein L7/L12 of *Neisseria gonorrhoeae*, was obtained in accordance with the method in Example 3.

### Example 8

### Reaction of monoclonal antibody that reacts with ribosomal protein L7/L12 of Neisseria gonorrhoeae, with Neisseria gonorrhoeae and other microorganisms

Monoclonal antibody was produced and recovered in accordance with standard methods using the positive hybridoma cells GCRB-3 obtained as previously described.

Basically, 5 x 10⁶ cells (in PBS) that had been subcultured using RPMI 1640 culture medium (containing 10 % FCS) were intraperitoneally injected into Balb/C mice that had been intraperitoneally injected with 0.5 ml Pristane 2 weeks earlier. Ascites was recovered 3 weeks later and the centrifugation supernatant was obtained.

The solution containing antibody that was obtained was adsorbed in a Protein A column (5 ml bed, Pharmacia) and washed with 3-bed volume of PBS. Then eluted with citrate buffer at pH 3, the antibody fraction was recovered and the monoclonal antibody that was produced by each hybridoma was obtained. The monoclonal antibody derived from the GCRB-3 hybridoma was used in ELISA.

The sandwich assay method was used to assess the monoclonal antibody. The monoclonal antibody that was prepared was used as an enzyme-labeled antibody by being chemically bound to peroxidase. That is, enzyme labeling was performed in accordance with the method in "Analytical Biochemistry" 132 (1983), 68-73 with the reagent S-acetylthioacetic acid N-hydroxysuccinimide for binding using horseradish peroxidase (Sigma Grade VI). By means of the ELISA reaction, a solution of commercial anti-*Neisseria gonorrhoeae* polyclonal antibody in PBS containing 0.05% sodium azide(Virostat, rabbit) was diluted to a concentration of 10 µg/ml and poured, 100 µl at a time, into a separate 96-well plate and adsorbed overnight at 4 °C.

After removal of the supernatant, 200 µl 1 % bovine serum albumin solution (in PBS) were added and reacted for 1 hour and blocked at room temperature. The supernatant was removed and the product was washed with washing solution (0.02 % Tween 20, PBS). One-hundred microliters of antigen solution, which had been obtained by adding Triton X-100 to culture solutions of each species of microorganisms to a concentration of 0.3 % and then extracting the solution for 5 minutes at room temperature, were added to this and reacted for 2 hours at room temperature. The supernatant was removed and the product was further washed with washing solution. Then 100 µl peroxidase-labeled anti-ribosomal protein L7/L12 antibody solution at 5 µg/ml were added and reacted for 1 hour at room temperature. The supernatant was removed and the product was washed with washing solution. TMB (KPL) solution was added, 100 µl at a time, and reacted for 20 minutes at room temperature. After coloration, 100 µl 1 N sulfuric acid were added to stop the reaction. Absorbance at 450 nm was determined.

As a result, as shown in Table 3 it is clear that when monoclonal antibody derived from hybridoma GCRB-3 was used as the enzyme-labeled antibody, all strains of *Neisseria gonorrhoeae* tested were detected at a sensitivity of 10⁶ cells/ml, while reactivity of other species belonging to the genus *Neisseria* and other microorganisms could not be detected, even at high concentrations of 10⁸ cells/ml and therefore, antibody with specific reactivity to *Neisseria gonorrhoeae* can be obtained by using monoclonal antibody to ribosomal protein L7/L12.

**Table 3**

| Results of Detection (10⁶ cells/ml) | | |
|---|---|---|
| *Neisseria gonorrhoeae* | ATCC9793 | + |
| *Neisseria gonorrhoeae* | ATCC19424 | + |
| *Neisseria gonorrhoeae* | ATCC27628 | + |
| *Neisseria gonorrhoeae* | ATCC27629 | + |
| *Neisseria gonorrhoeae* | ATCC27630 | + |
| *Neisseria gonorrhoeae* | ATCC27631 | + |
| *Neisseria gonorrhoeae* | ATCC27632 | + |
| *Neisseria gonorrhoeae* | ATCC27633 | + |
| *Neisseria gonorrhoeae* | ATCC35541 | + |
| *Neisseria gonorrhoeae* | ATCC35542 | + |
| *Neisseria gonorrhoeae* | ATCC43069 | + |
| *Neisseria gonorrhoeae* | ATCC43070 | + |
| *Neisseria gonorrhoeae* | ATCC49226 | + |

| Results of Detection (10⁸ cells/ml) | | |
|---|---|---|
| *Neisseria meningitidis* | ATCC13090 | - |
| *Neisseria lactamica* | ATCC30011 | - |
| *Neisseria mucosa* | ATCC35611 | - |
| *Neisseria sicca* | ATCC9913 | - |
| | | |
| *Branhamella catarrharis* | ATCC25240 | - |
| *Haemophilus influenzae* | ATCC10211 | - |
| *Escherichia coli* | ATCC25922 | - |
| *Klebsiella pneumoniae* | ATCC13883 | - |
| (+: Positive; -: Negative) | | |

### Example 9

### Acquisition of ant-ribosomal protein L7/L12 monoclonal antibody specific to genus Neisseria

After inoculating an appropriate amount of *Neisseria gonorrhoeae* strain IID821 (obtained from Tokyo University School of Medicine Laboratories) in a chocolate agar culture medium, the strain was cultivated for 24 hours in a CO₂ incubator under conditions of 37 °C and 5.0 % CO₂. The colonies that grew were suspended in a TE buffer to a final concentration of approximately 5 x 10⁹ CFU/ml. Approximately 1.5 ml of this suspension was transferred to a microcentrifugation tube and centrifuged for 2 minutes at 10,000 rpm. The supernatant was discarded. The sediment was resuspended in 567 µl TE buffer. Then 30 µl 10 % SDS and 3 µl 20 mg/ml Proteinase K solution were added and thoroughly mixed. The suspension was incubated for another hour at 37 °C. Next, after adding 80 µl 10 % cetyl trimethyl ammonium bromide/0.7 M NaCl solution and thoroughly mixing the product, it was incubated for 10 minutes at 65 °C. Next, 700 µl chloroform-isoamyl alcohol solution at a volume ratio of 24:1 was added and stirred well.

The solution was centrifuged for 5 minutes (while being kept at 4 °C) at 12,000 rpm using a microcentrifugation device and the aqueous fraction was transferred to a new microtube. Isopropanol was added to the fraction at 0.6-times its volume and the tube was vigorously shaken to form sediment of the DNA. The white DNA sediment was scooped with a glass rod and transferred to a different microcentrifugation tube containing 1 ml 70 % ethanol (cooled to -20 °C).

Next, the product was centrifuged for 5 minutes at 10,000 rpm and the supernatant was gently removed. Then another 1 ml 70 % ethanol was added and the product was centrifuged for 5 more minutes. Once the supernatant had been removed, the sediment was dissolved in 100 µl TE buffer to obtain the DNA solution. The concentration of the genomic DNA solution was determined quantitatively in accordance with E5, Spectrophotometric determination of the amount of DNA or RNA, "Molecular cloning: A laboratory manual," 1989, Eds. Sambrook, J., Fritsch, E.F., and Maniatis, T., Cold Spring Harbor Laboratory Press.

PCR was performed using 10 ng of this genomic DNA. Taq polymerase (Takara Co., Ltd., code R001A) was employed for PCR. Then, 5 µl buffer attached to enzyme, 4 µl dNTP mixture attached to enzyme, and 200 pmol each of synthetic oligonucleotide E shown in Sequence No. 15 of the Sequence Table and synthetic oligonucleotide F shown in Sequence No. 16 of the Sequence Table, which were designed based on the ribosomal protein L7/L12 DNA sequence of *Neisseria gonorrhoeae* acquired from Internet information (Oklahoma University, *N. Gonorrhoeae* Genome Project, disclosed genome DNA data) because of the similarity with ribosomal protein L7/L12 DNA sequence of other bacteria, were added to the enzyme to bring the final volume to 50 µl.

This mixture was cycled 5 times with a TaKaRa PCR Thermal Cycler 480 for 1 minute at 95 °C, 2 minutes at 50 °C, and 3 minutes at 72 °C and was then cycled 25 times for 1 minute at 95 °C, 2 minutes at 60 °C, and 3 minutes at 72 °C. Electrophoresis was performed in 1.5 % agarose gel using some of this PCR product. This product was then stained with ethidium bromide (Nihon Gene Co., ltd.) and observed under ultraviolet rays to confirm amplification of approximately 400 bp CDNA. After digestion treatment with restriction endonucleases BamHI and XhoI, electrophoresis was performed in 1.5 % agarose gel and staining with ethidium bromide was carried out. An approximately 370 bp band was cut out from the gel. This band was purified with Suprec01 (Takara Co., Ltd.) and then inserted into pGEX-6P-1 (Pharmacia), which is a commercial vector. This same vector can function as an expression vector for the desired molecule, which can express fused protein with GST protein, by insertion of the desired gene fragment into the appropriate restriction endonuclease site. Actually, vector pGEX-6P-1 and the previous DNA were mixed together at a molar ratio of 1:5 and DNA was inserted into the vector with T4 DNA ligase (Invitrogen Co.). vector pGEX-6P-1 into which DNA had been inserted was genetically introduced to *Escherichia coli* One-Shot Competent Cells (Invitrogen Co., Ltd.) and then inoculated in a plate of L-Broth (Takara Co., ltd.) semi-sold culture plate containing 50 µg/ml ampicillin (Sigma). The plate was then set aside at 37 °C for 12 hours and the colonies that grew were selected at random and inoculated into 2 ml L-Broth liquid culture medium containing the same concentration of ampicillin. Shake cultivation was performed at 37 °C for 8 hours and the bacteria was recovered and the plasmid was separated using Wizard Miniprep in accordance with the attached literature. The plasmid was cleaved with restriction endonuclease BamHI/XhoI. Insertion of said PCR product was confirmed by cutting out approximately 370 bp DNA. The base sequence of the DNA that had been inserted was determined using said clone.

Determination of the base sequence of the inserted DNA fragment was performed using the Fluorescence Sequencer of Applied Biosystems. The sequence sample was prepared using PRISM, Ready Reaction Dye Terminator Cycle Sequencing Kit (Applied Biosystems). First, 9.5 µl reaction stock solution, 4.0 µl T7 promoter primer at 0.8 pmol/µl (Gibco BRL) and 6.5 µl template DNA for sequencing at 0.1-6 µg/µl were added to a microtube with a capacity of 0.5 ml and mixed. After layering with 100 µl mineral oil, PCR amplification was performed for 25 cycles, where one cycle consisted of 30 seconds at 96 °C, 15 seconds at 55 °C, and 4 minutes at 60 °C. The product was then kept at 4 °C for 5 minutes. After the reaction was completed, 80 µl sterilized pure water was added and stirred. The product was centrifuged and the aqueous layer was extracted 3 times with phenol-chloroform. Ten microliters 3 M sodium acetate (pH 5.2) and 300 µl ethanol were added to 100 µl the aqueous layer and stirred. The product was then centrifuged for 15 minutes at room temperature and 14000 rpm and the sediment was recovered. Once the sediment was washed with 75 % ethanol, it was dried under a vacuum for 2 minutes to obtain the sequencing sample. The sequencing sample was dissolved in formamide containing 4 µl 10 mM EDTA and denatured for 2 minutes at 90 °C. This was then cooled in ice and submitted to sequencing.

One of the 4 clones obtained had homology of the sequence with the probe used for PCR. In addition, DNA sequences extremely similar to the gene sequence of ribosomal protein L7/L12 gene of the other microorganisms, for example, *Haemophilus influenzae*, were discovered. The entire base sequence and the corresponding amino acid sequence of the structural gene moiety are as shown in Sequence ID No. 21 and No. 22 of the Sequence Table. This gene fragment clearly codes for *Neisseria gonorrhoeae* ribosomal protein L7/L12.

50 ml *Escherichia coli* into which expression vector had been inserted was cultivated overnight in a two-fold concentration YT medium at 37 °C. Then, 450ml of the two-fold concentration YT medium was heated at 37 °C for 1 hour. 50 ml of the *Escherichia coli* solution that had been cultivated overnight was introduced to 450ml of the aforementioned medium. After cultivation for one hour at 37 °C, 100 µl 500 mM IPTG was introduced and cultivated for 4 hours at 25 °C. The product was then recovered and introduced 250 ml each to a centrifugation tube and centrifuged for 20 minutes at 5000 rpm. The supernatant was discarded and dissolved in 25 ml each 50 mM Tris-HCl at a pH of 7.4 and Lysis buffer containing 25 % sucrose.

Furthermore, 1.25 ml 10% NP-40 and 125 µl 1 M MgCl₂ were added and transferred to a plastic tube. Sonication was performed 1 minute x 5 times while ice cold. The product was centrifuged for 15 minutes at 12,000 rpm and the supernatant was recovered.

Next, the aforementioned supernatant was adsorbed on a glutathione sepharose column (manufactured by Pharmacia) conditioned with PBS. Then, the column was washed with PBS three times the bed volume. Elution was performed with 50 mM Tris-HCl at a pH of 8.0 containing 10 mM glutathione. The protein content in the fraction was determined by the pigment bonding method (Bradford method; BioRad Co.) and the main fraction was acquired. The main fraction was dialyzed three times against 3L PBS.

1 ml of a cleavage buffer containing 500 mM Tris-HCl (pH 7.0), 1.5 M NaCl, 10 mM EDTA, and 10 mM DTT was added to 10 ml of 1 mg/ml solution of the resulting GST fusion ribosomal protein L7/L12. 100 µl of 2 u/µl PreScission Protease (manufactured by Pharmacia company) was further added and reacted at 4 °C to separate the GST moiety from ribosomal protein L7/L12.

The reaction solution was passed through a glutathione sepharose column which had been conditioned with PBS. The solution coming out from the column was recovered. One bed volume of PBS was passed through and also recovered. Purity of the purified ribosomal protein L7/L12 that was obtained was confirmed by electrophoresis to be approximately 90 %, showing that a purity satisfactory for an immunogen could be guaranteed.

First, 100 µg protein antigen of ribosomal protein L7/L12 of *Neisseria gonorrhoeae* was dissolved in 200 µl PBS and then 200 µl Freund's complete adjuvant was added and mixed and emulsification was performed. 200 µl was intraperitoneally injected to immunize mice. Then, the same emulsion antigen was intraperitoneally injected after 2 weeks, after 4 weeks, and after 6 weeks. A two-fold concentration antigen emulsion was further injected intraperitoneally after 10 weeks and after 14 weeks. The spleen was excised 3 days after the final immunization and submitted to cell fusion.

After thoroughly mixing 2 x 10⁷ myeloma cells per 10⁸ spleen cells from mice, which had been recovered aseptically, in a glass tube, the mixture was centrifuged for 5 minutes at 1500 rpm and the supernatant was discarded. The cells were thoroughly mixed.

The myeloma cells used for cell fusion were obtained by cultivation of cell strain NS-1 with an RPMI 1640 culture medium containing 10 % FCS, cultivating this product beginning 2 weeks before cell fusion using an RPMI 1640 medium containing 0.13 mM azaguanine, 0.5 µg/ml MC-210, and 10 % FCS for 1 weeks, and then further cultivating the cell strain for 1 week with an RPMI 1640 medium containing 10 % FCS. 30 ml of RPMI 1640 culture medium 50 ml that had been kept at 37°C was added to the mixed cell sample and centrifuged at 1,500 rpm. After removal of the supernatant, 1 ml 50 % polyethylene glycol that had been kept at 37 °C was added and stirred for 2 minute. 10 ml RPMI 1640 medium kept at 37 °C was added and the solution was vigorously mixed for approximately 5 minutes as it was suctioned and evacuated from a sterile pipette.

After centrifugation for 5 minutes at 1,000 rpm and removal of the supernatant, 30 ml HAT culture medium were added to bring the cell concentration to x 10⁶ cells/ml. This mixture was stirred till uniform and then poured, 0.1 ml at a time, into a 96-well culture plate and cultivated at 37 °C under 7 % CO₂. HAT medium was added, 0.1 ml at a time, on Day 1 and at Week 1 and Week 2.

Then the cells that had produced the desired antibody were assessed by ELISA. Solutions of ribosomal protein L7/L12 of *Neisseria gonorrhoeae* dissolved in PBS containing 0.05 % sodium azide were diluted to 10 µg/ml and were poured, 100 µl at a time, into separate 96-well plates and adsorbed overnight at 4 °C. After removal of the supernatant, 200 µl 1 % bovine serum albumin solution (in PBS) were added and reacted and blocked for 1 hour at room temperature. The supernatant was removed and the product was washed with a washing solution (0.02 % Tween 20, PBS), 100 µl of a culture solution of fusion cells was added and reacted for two hours at room temperature. The supernatant was removed and the product was further washed with a washing solution. Then, 100 µl of a peroxidase-labeled goat anti-mouse antibody solution at 50 ng/ml was added and reacted for one hour at room temperature. The supernatant was removed and the product was washed with a washing solution. TMB (KPL) solution was added, 100 µl at a time, and reacted for 20 minutes at room temperature. After coloration, 100 µl 1 N sulfuric acid were added to stop the reaction. Absorbance at 450 nm was determined.

As a result, positive wells that reacted with ribosomal protein L7/L12 were detected, confirming presence of the antibody to ribosomal protein L7/L12.

Therefore, the cells in the positive wells were recovered and cultivated with HAT medium in a 24-well plastic plate. The fused medium that had been cultivated was diluted with an HT medium to a cell count of approximately 20 cells/ml. Then 50 µl the medium was mixed with 10⁶ six-week-old mouse thyroid cells suspended in the HT medium in a 96-well culture plate. The cells were cultivated for 2 weeks at 37 °C under the conditions of 7 % CO₂. The antibody titer in the culture supernatant was determined by the aforementioned ELISA method and, the cells those showed a positive reaction with ribosomal protein L7/L12 were recovered. Furthermore, the same dilution and cloning procedure was repeated to obtain a total of 4 clones of hybridoma AMGC-5 to AMGC-8.

Monoclonal antibody was produced and recovered according to standard methods, using the positive hybridoma cells obtained as previously described.

Specifically, cells subcultured in RPMI 1640 medium (containing 10 % FCS) was diluted in serum-free medium to about 2 x 10⁵ cells/ml, 3.3 x 10⁵ cells/ml, and 5 x 10⁵ cells/ml in 25 cm² culture flask, and the total was made 5 ml. After cells were grown for 3 to 5 days in 7 % CO₂ at 37 °C, flask that contains the least number of original cells was selected among flasks in which cells were grown. The same procedure was repeated until the cells diluted to 2 x 10⁵ cells/ml grow to 2 x 10⁶ cells/ml in 3 to 4 days, thereby adapting the cells with serum-free medium. Next, cloning was performed in a 96-well plate for bacterial culture to select cells exhibiting fastest growth and a highest antibody titer. The selected cells were grown in a 24-well plate and diluted with a serum-free medium in a 25 cm² culture flask to a concentration of about 2 x 10⁵ cells/ml and the total volume was made to 10 ml. After incubation for 3 to 4 days in 7 % CO₂ at 37 °C to a concentration of 1 x 10⁶ cells/ml, the culture broth 100 ml, 1 x 10⁶ cells/ml was transferred to a bottle for mass cultivation which were grown in the same manner in a 75 cm² flask. 100 ml of a serum-free medium was added to the mixture and was incubated at 37 °C for two days while stirring. 200 ml of the serum-free medium was added again and the mixture was incubated for a further two days. The culture broth was divided into four aliquots, one volume serum-free medium was added to each portion, followed by incubation for two days. After further addition of 400 ml of the serum-free medium, the culture broth was incubated for 6 days. The culture broth was collected and centrifuged at 10,000 rpm for 15 minutes to obtain culture supernatant containing the target antibody. After adding sodium azide to final concentration 0.1 %, the culture supernatant was stored at 4 °C, 100 ml of the supernatant containing the antibody was diluted 5-fold with PBS and adsorbed in a protein G column (5 ml bed, Pharmacia) and washed with 3-bed volume of PBS. Then eluted with citrate buffer at pH 3, the antibody fraction was recovered and monoclonal antibody produced by each hybridoma was obtained.

The monoclonal antibodies originating from the four hybridoma clones were evaluated according to the OIA method described in International Patent Application Japanese Laid-open No. 509565/1995.

Specifically, the OIA method comprises preparing a reactive substrate by reacting an antibody for capture on a silicon wafer having a thin film layer of silicon nitride, causing this substrate to react with an antigen which is an extract of microorganisms for a prescribed period of time, causing the captured antigen to react with an antibody (an amplification reagent) which is an enzyme-labeled antibody, and finally adding a substrate solution to produce a thin-film precipitate. The antigen-antibody reaction can be judged visually by a degree of light interference color produced in the precipitate.

The monoclonal antibody preparation was used as a capture antibody to be immobilized on a silicon wafer having a silicon nitride thin film layer in the OIA method. Moreover, peroxidase-labeled AMGC-1 monoclonal antibody which can non-specifically react with ribosomal proteins L7/L12 protein of a variety of microorganisms described in Reference Example was used as the detect antibody. That is, enzyme labeling was performed in accordance with the method in "Analytical Biochemistry" 132 (1983), 68-73 with the reagent S-acetylthioacetic acid N-hydroxysuccinimide for binding using horseradish peroxidase (Sigma Grade VI).

In the OIA reaction, monoclonal antibody in a PBS containing 0.05 % sodium azide was diluted with 0.1 M HEPES (pH 8.0) to a concentration of 10 µg/ml and added onto a silicone wafer which has a thin film layer of silicon nitride, 50 µl at a time, to react for 30 minutes at room temperature, followed by washing with distilled water and use. 15 µl of antigen solution, which had been obtained by adding 0.5 % Triton X-100 to culture suspension of various species of microorganisms and then extracting the suspension for 5 minutes at room temperature, was added to the specimen obtained in the above-described procedure and reacted for 10 minutes at room temperature. Then, 15 µl of 20 µg/ml peroxidase-labeled AMGC1 was added and reacted for 10 minutes. After washing with distilled water, a substrate solution (KPL Co.) was added, 15 µl at a time, and reacted for 5 minutes at room temperature. The product was washed with distilled water to judge the concentration of detection signals as an intensity of light interference by naked eyes.

As a result, as shown in Table 4 it is clear that when monoclonal antibody derived from hybridoma AMGC-8 was used as the capture antibody, all strains of *Neisseria* genus tested were detected at a sensitivity of 10⁸ cells/ml, while reactivity of other microorganisms could not be detected. Thus, the antibody with specific reactivity to *Neisseria* genus was confirmed to have been obtained by using the monoclonal antibody to ribosomal protein L7/L12.

**Table 4**

| | Results of Detection (10⁸ cells/ml) | |
|---|---|---|
| *Neisseria gonorrhoeae* | IID821 | + |
| *Neisseria lactamica* | ATCC23970 | + |
| *Neisseria meningitidis* | ATCC13090 | + |
| | | |
| *Escherichia coli* | ATCC25922 | - |
| *Enterococcus faecalis* | ATCC19433 | - |
| *Haemophilus influenzae* | ATCC10211 | - |
| *Klebsiella pneumoniae* | ATCC13883 | - |
| Pseudomonas aeruginosa | ATCC27853 | - |
| *GroupB streptococcus* | ATCC12386 | - |
| *Staphylococcus aureus* | ATCC25923 | - |
| *Streptococcus pneumoniae* | ATCC27336 | - |
| *Streptococcus pyogenes* | ATCC19615 | - |
| (+: Positive; -: Negative) | | |

### Example 10

### Acquisition of a polyclonal antibody which specifically reacts with ribosomal protein L7/L12 of Haemophilus influenzae using a ribosomal protein L7/L12 protein-immobilized affinity column

A centrifugal supernatant of *Haemophilus influenzae* cell extract, which had been treated with 0.5% Triton X-100 was used as an antigen. About 1.2 ml of a physiological saline solution containing 100 µg of antigen was emulsified with the addition of 1.5 ml of Freund's adjuvant. The emulsion was subcutaneously injected into four SPF Japanese White Rabbits to immunize the animals. The rabbits were immunized 5 to 6 times, once every two weeks, and the antibody titer was confirmed.

The antibody titer was confirmed by the ELISA method. Solutions of ribosomal protein L7/L12 of *Haemophilus influenzae* dissolved in PBS containing 0.05 % sodium azide diluted to 10 µg/ml were poured, 100 µl at a time, into 96-well plates and adsorbed overnight at 4 °C. After removal of the supernatant, 200 µl 1 % bovine serum albumin solution (in PBS) were added and reacted and blocked for 1 hour at room temperature. The supernatant was removed and the product was washed with a washing solution (0.02 % Tween 20, PBS). 100 µl of a solution obtained by diluting normal rabbit serum and immunized rabbit antiserum was added and reacted for two hours at room temperature. The supernatant was removed and the product was further washed with a washing solution. Then, 100 µl of a peroxidase-labeled goat anti-rabbit IgG antibody solution at 50 ng/ml was added and reacted for one hour at room temperature. The supernatant was removed and the product was washed with a washing solution. OPD solution (Sigma Co.) was added, 100 µl at a time, and reacted for 20 minutes at room temperature. After coloration, 100 µl of 1 N sulfuric acid was added to stop the reaction. Absorbance at 492 nm was determined.

After confirming that the antibody titer had increased, a large quantity of blood was collected. Blood was collected in a centrifugal tube made of glass from the ear artery, allowed to stand for one hour at 37 °C, then overnight at 4 °C. The mixture was centrifuged at 3000 rpm for 5 minutes and the supernatant was recovered. The resulting 4 lots of anti-serum were stored at 4 °C.

Next, an affinity column with immobilized ribosomal protein L7/L12 of *Haemophilus influenzae* and *Neisseria gonorrhoeae* was prepared. HiTrap NHS-activated column (1 ml, manufactured by Pharmacia) was used. Immediately after replacing the column with 6 ml of 1 mM HCl, 1 ml of a PBS solution of ribosomal protein L7/L12 adjusted to 1 mg/ml was charged. The column was allowed to stand for 15 minutes at 25 °C. This procedure was repeated 5 times, thereby feeding the total 5 ml of the PBS solution of ribosomal protein L7/L12. Then, 6 ml of Buffer A (0.5 M ethanolamine, 0.5 M NaCl, pH 8.3), 6 ml of Buffer B (0.1 M acetic acid, 0.5 M NaCl, pH 4), and 6 ml of Buffer A were charged as blocking reagents. After allowing to stand for 15 minutes at 25 °C, 6 ml of Buffer B, 6 ml of Buffer A, and 6 ml of Buffer B were further added. The mixture was then equilibrated with 6 ml of PBS.

Using the affinity column with immobilized ribosomal protein L7/L12 of *Haemophilus influenzae*, the polyclonal antibody in the resulting anti-serum was purified using the supernatant of Triton X-100 treated bacteria of *Haemophilus influenzae* as an antigen. This antiserum was first diluted with PBS to a volume of 5 times, passed through a 0.45 µm filter, then caused to be adsorbed in the column immobilized with ribosomal protein L7/L12 of *Haemophilus influenzae* at a flow rate of 0.5 ml/min. After elution from the column with 0.1 M glycine (pH 2.1) and immediately neutralizing with 1 M Tris-HCl (pH 9.0), eluted fractions of the target antibody were recovered by the ELISA method, the same as anti-titer measuring method. These fractions were passed through the affinity column immobilized with the ribosomal protein L7/L12 of *Neisseria gonorrhoeae*, which was equilibrated with PBS whereby the antibody that reacts with the ribosomal protein L7/L12 of *Neisseria gonorrhoeae* was adsorbed and fraction which passed through without adsorption was recovered.

The polyclonal antibody purified in this manner was evaluated by the same OIA method as in Example 6.

The purified antibody was used as a capture antibody for the OIA method. Moreover, peroxidase-labeled AMGC-1 monoclonal antibody described in Reference Example was used as the detect antibody. That is, enzyme labeling was performed in accordance with the method in "Analytical Biochemistry" 132 (1983), 68-73 with the reagent S-acetylthioacetic acid N-hydroxysuccinimide for binding using horseradish peroxidase (Sigma Grade VI).

In the OIA reaction, the purified polyclonal antibody in a PBS containing 0.05 % sodium azide was diluted with 0.1 M HEPES (pH 8.0) to a concentration of 10 µg/ml and added onto a silicone wafer, 50 µl at a time, to react for 30 minutes at room temperature, followed by washing with distilled water and use.

15 µl of antigen solution, which had been obtained by adding 0.5 % Triton X-100 to culture suspension of various species of microorganisms and then extracting the suspension for 5 minutes at room temperature, was added to the specimen obtained in the above-described procedure and reacted for 10 minutes at room temperature. Then, 15 µl of 20 µg/ml peroxidase-labeled AMGC1 was added and reacted for 10 minutes. After washing with distilled water, a substrate solution (KPL) was added, 15 µl at a time, and reacted for 5 minutes at room temperature. The product was washed with distilled water to observe the blue color concentration by the naked eyes.

As a result, as shown in Table 5 it is clear that when the purified polyclonal antibody of APHI2-2 was used as the capture antibody, *Haemophilus influenzae* tested at a sensitivity of 10⁸ bacteria/ml was detected, while reactivity of other microorganisms could not be detected. Thus, the antibody with specific reactivity to *Haemophilus influenzae* was confirmed to have been obtained by using the polyclonal antibody purified by an affinity column immobilized with the ribosomal protein L7/L12 of *Haemophilus influenzae*.

**Table 5**

| Results of Detection (10⁸ cells/ml) | | |
|---|---|---|
| *Haemophilus influenzae* | ATCC10211 | + |
| | | |
| *Escherichia coli* | ATCC25922 | - |
| *Enterococcus faecalis* | ATCC19433 | - |
| *Klebsiella pneumoniae* | ATCC13883 | - |
| *Neisseria gonorrhoeae* | IID821 | - |
| *Neisseria lactamica* | ATCC23970 | - |
| *Neisseria meningitidis* | ATCC13090 | - |
| *Pseudomonas aeruginosa* | ATCC27853 | - |
| *GroupB Streptococcus* | ATCC12386 | - |
| *Staphylococcus aureus* | ATCC25923 | - |
| *Streptococcus pneumoniae* | ATCC27336 | - |
| *Streptococcus pyogenes* | ATCC19615 | - |
| (+: Positive; -: Negative) | | |

### Reference Example 1

### Acquisition of monoclonal antibody that reacts non-specifically with ribosomal protein L7/L12 of various bacteria

So called sandwich assay in which an antigen is sandwiched between a capture antibody and a labeled antibody for detection is useful to detect microorganisms by optical immunoassay and the ELISA method because of its high detection sensitivity. In this instance, not only an antibody which specifically reacts with antigens originating from the subject microorganism, but also another antibody which recognizes an antigen epitope different from that of the specific antibody are required.

Antibodies which react non-specifically with ribosomal protein L7/L12 originating various microorganisms are very useful as an antibody which can constitute a sandwich assay with an antibody which reacts specifically with ribosomal protein L7/L12.

Fortunately, ribosomal proteins L7/L12 protein of a variety of microorganisms have a region wherein the amino acid sequence is homologous. Here, the inventors have been successful in acquiring a monoclonal antibody which exhibits a cross reaction with ribosomal proteins L7/L12 protein of various species of microorganisms from *Neisseria gonorrhoeae*. It has been discovered that an antibody to anti-ribosomal protein L7/L12 having no specificity, which was acquired from one species of microorganisms can be used for sandwich assay of several kinds of microorganisms.

Cloning of ribosomal protein L7/L12 genes from *Neisseria gonorrhoeae*, mass expression in Escherichia coli and purification of the same protein, and preparation of monoclonal antibody to the same protein were performed.

After inoculating an appropriate amount of *Neisseria gonorrhoeae* strain IID821 (obtained from Tokyo University School of Medicine Laboratories) in a chocolate agar culture medium, the strain was cultivated for 24 hours in a CO₂ incubator under conditions of 37 °C and 5.0 % CO₂. The colonies that grew were suspended in a TE buffer to a final concentration of approximately 5 x 10⁹ CFU/ml. Approximately 1.5 ml of this suspension was transferred to a microcentrifugation tube and centrifuged for 2 minutes at 10,000 rpm. The supernatant was discarded. The sediment was resuspended in 567 µl TE buffer. Then 30 µl 10% SDS and 3 µl 20 mg/ml Proteinase K solution were added and thoroughly mixed. The suspension was incubated for another hour at 37 °C. Next, after adding 80 µl 10 % cetyl trimethyl ammonium bromide/0.7 M NaCl solution and thoroughly mixing the product, it was incubated for 10 minutes at 65 °C. Next, 700 µl chloroform-isoamyl alcohol solution at a volume ratio of 24:1 was added and stirred well. The solution was centrifuged for 5 minutes (while being kept at 4 °C) at 12,000 rpm using a microcentrifugation device and the aqueous fraction was transferred to a new microtube. Isopropanol was added to the fraction at 0.6-times its volume and the tube was vigorously shaken to form sediment of the DNA. The white DNA sediment was scooped with a glass rod and transferred to a different microcentrifugation tube containing 1 ml 70 % ethanol (cooled to -20 °C).

Next, the product was centrifuged for 5 minutes at 10,000 rpm and the supernatant was gently removed. Then another 1 ml 70 % ethanol was added and the product was centrifuged for 5 more minutes. Once the supernatant had been removed, the sediment was dissolved in 100 µl TE buffer to obtain the DNA solution. The concentration of the genomic DNA solution was determined quantitatively in accordance with E5, Spectrophotometric determination of the amount of DNA or RNA, "Molecular cloning: A laboratory manual,"1989, Eds. Sambrook, J., Fritsch, E.F., and Maniatis, T., Cold Spring Harbor Laboratory Press.

PCR was performed using 10 ng of this genomic DNA. Taq polymerase (Takara Co., Ltd., code R001A) was employed for PCR. Then, 5 µl of a buffer attached to enzyme, 4 µl of dNTP mixture attached to enzyme, and 200 pmol of each of synthetic oligonucleotide E shown in Sequence No. 15 of the Sequence Table and synthetic oligonucleotide F shown . in Sequence No. 16 of the Sequence Table, which were designed based on the ribosomal protein L7/L12 DNA sequence of *Neisseria gonorrhoeae* acquired from Internet information (Oklahoma University, *N. Gonorrhoeae* Genome Project, disclosed genomic DNA data) because of the similarity with ribosomal protein L7/L12 DNA sequence of other bacteria, were added to the enzyme to bring the final volume to 50 µl.

This mixture was cycled 5 times with a TaKaRa PCR Thermal Cycler 480 for 1 minute at 95 °C, 2 minutes at 50 °C, and 3 minutes at 72 °C and was then cycled 25 times for 1 minute at 95 °C, 2 minutes at 60 °C, and 3 minutes at 72 °C. Electrophoresis was performed in 1.5 % agarose gel using some of this PCR product. This product was then stained with ethidium bromide (Nihon Gene Co., ltd.) and observed under ultraviolet rays to confirm amplification of approximately 400 bp cDNA. After digestion treatment with restriction endonucleases BamHI and XhoI, electrophoresis was performed in 1.5 % agarose gel and staining with ethidium bromide was carried out. An approximately 370 bp band was cut out from the gel. This band was purified with Suprec01 (Takara Co., Ltd.) and then inserted into pGEX-4T-1 (Pharmacia), which is a commercial vector. Actually, vector pGEX-4T-1 and the previous DNA were mixed together at a molar ratio of 1:3 and DNA was inserted into the vector with T4 DNA ligase (Invitrogen Co.). Vector pGEX-4T-1 into which DNA had been inserted was genetically introduced to *Escherichia coli* One-Shot Competent Cells (Invitrogen Co., Ltd.) and then inoculated in a plate of L-Broth (Takara Co., Ltd.) semi-sold culture plate containing 50 µg/ml ampicillin (Sigma). The plate was then set aside at 37 °C for 12 hours and the colonies that grew were selected at random and inoculated into 2 ml L-Broth liquid culture medium containing the same concentration of ampicillin. Shake cultivation was performed at 37 °C for 8 hours and the bacteria was recovered and the plasmid was separated using Wizard Miniprep in accordance with the attached literature. The plasmid was cleaved with restriction endonuclease BamHI/XhoI. Insertion of said PCR product was confirmed by cutting out approximately 370 bp DNA. The base sequence of the DNA that had been inserted was determined using said clone.

Determination of the base sequence of the inserted DNA fragment was performed using the Fluorescence Sequencer of Applied Biosystems. The sequence sample was prepared using PRISM, Ready Reaction Dye Terminator Cycle Sequencing Kit (Applied Biosystems). First, 9.5 µl reaction stock solution, 4.0 µl T7 promoter primer at 0.8 pmol/µl (Gibco BRL) and 6.5 µl template DNA for sequencing at 0.16 µg/µl were added to a microtube with a capacity of 0.5 ml and mixed. After superposition with 100 µl mineral oil, PCR amplification was performed for 25 cycles, where one cycle consisted of 30 seconds at 96 °C, 15 seconds at 55 °C, and 5 minutes at 60 °C. The product was then kept at 4 °C for 4 minutes. After the reaction was completed, 80 µl sterilized pure water was added and stirred. The product was centrifuged and the aqueous layer was extracted 3 times with phenol-chloroform. Ten microliters 3 M sodium acetate (pH 5.2) and 300 µl ethanol were added to 100 µl aqueous layer and stirred. The product was then centrifuged for 15 minutes at room temperature and 14,000 rpm and the sediment was recovered. Once the sediment was washed with 75 % ethanol, it was dried under a vacuum for 2 minutes to obtain the sequencing sample. The sequencing sample was dissolved in formamide containing 4 µl 10 mM EDTA and denatured for 2 minutes at 90 °C. This was then cooled in ice and submitted to sequencing. One of the 5 clones obtained had homology of the sequence with the probe used for PCR. In addition, DNA sequences extremely similar to the gene sequence of ribosomal protein L7/L12 gene of the other microorganisms, for example, *Haemophilus influenzae*, were discovered. The entire base sequence and the corresponding amino acid sequence of the structural gene moiety are as shown in Sequence No. 21 and No. 22 of the Sequence Table. This gene fragment clearly codes for *Neisseria gonorrhoeae* ribosomal protein L7/L12.

*Neisseria gonorrhoeae* GST fused ribosome protein L7/L12 prepared by the same method as in Example 2 was obtained using the *Neisseria gonorrhoeae* GST fusion ribosomal protein L7/L12 expression vector constructed in this way. Furthermore, hybridoma strain AMGC1, which produces monoclonal antibody to ribosomal protein L7/L12 of *Neisseria gonorrhoeae*, was obtained in accordance with the method similar to the method of Example 3. Monoclonal antibody was produced and recovered in accordance with standard methods using the positive hybridoma cells of AMGC1 strain obtained as previously described.

Specifically, cells subcultured in RPMI 1640 medium (containing 10 % FCS) was diluted with a serum-free medium to about 2 x 10⁵ cells/ml, 3.3 x 10⁵ cells/ml, and 5 x 10⁵ cells/ml in 25 cm² culture flasks, and the total was made 5 ml. After cell were grown for 3 to 5 days in 7 % CO₂ at 37 °C, a flask which contains the least number of original cells was selected among flasks in which cells were grown. The same procedure was repeated until the cells diluted to 2 x 10⁵ cells/ml grow to 2 x 10⁶ cells/ml in 3 to 4 days, thereby adapting the cells with the serum-free medium. Next, cloning was performed in a 96-well plate for bacteria cultivation to select cells exhibiting fastest growth and a highest antibody titer. The selected cells were grown in a 24-well plate and diluted with a serum-free medium in a 25 cm² culture flask to a concentration of about 2 x 10⁵ cells/ml and the total volume was made 10 ml. After incubation for 3 to 4 days in 7 % CO₂ at 37 °C to a concentration of 1 x 10⁶ cells/ml, the culture broth 100 ml, 1 x 10⁶ cells/ml was transferred to a bottle for mass cultivation which were grown in the same manner in a 75 cm² flask. 100 ml of a serum-free medium was added to the mixture, which was incubated at 37 °C for two days while stirring. 200 ml of the serum-free medium was added again and the mixture was incubated for a further two days. The culture broth was divided into four aliquots, the serum-free medium was added to each portion, followed by incubation for two days. After further addition of 400 ml of the serum-free medium, the culture broth was incubated for 6 days. The culture broth was collected and centrifuged at 10,000 rpm for 15 minutes to obtain a culture supernatant including the target antibody. After the addition of sodium azide to final concentration 0.1 %, the culture supernatant was stored at 4 °C. 100 ml of the solution containing the antibody that was obtained was diluted 5-fold with PBS and adsorbed in a Protein A column (5 ml bed volume, Pharmacia) and washed with 3-bed volume of PBS. Then eluted with citrate buffer at pH 3, the antibody fraction was recovered and monoclonal antibody produced by each hybridoma was obtained. The monoclonal antibody derived from the hybridoma was used in ELISA.

To evaluate the antibody, 96-well plates sensitized with ribosomal proteins L7/L12 of various microorganisms were used as an antigen. The monoclonal antibody prepared was reacted, followed by the reaction of horseradish peroxidase-labeled anti-mouse IgG (manufactured by MBL, Code 330) as a secondary antigen, and finally the antibody was detected using an enzyme reaction coloring reagent. In the ELISA reaction, solutions of recombinant ribosomal protein L7/L12 of *Neisseria gonorrhoeae*, *Haemophilus influenzae*, and *Streptococcus pneumoniae* dissolved in PBS containing 0.05 % sodium azide diluted to 1 µg/ml were poured, 100 µl at a time, into separate 96-well plates and adsorbed overnight at 4 °C. After removal of the supernatant, 200 µl 1% bovine serum albumin solution (in PBS) were added and reacted and blocked for 1 hour at room temperature. The supernatant was removed and the product was washed with a washing solution (0.02 % Tween 20, PBS). Solutions of AMGC1 antibody, at concentrations of 0.1-1 µg/ml, in an amount of 100 µl each, were added and reacted for two hours at room temperature. The supernatant was removed and the product was further washed with a washing solution. Then, 100 µl of a solution of 5 µg/ml horse radish peroxidase-labeled anti-mouse IgG (manufactured by MBL, Code 330) was added and reacted for one hour at room temperature. The supernatant was removed and the product was washed with a washing solution. TMB (KPL) solution was added, 100 µl at a time, and reacted for 20 minutes at room temperature. After coloration, 100 µl 1 N sulfuric acid were added to stop the reaction. Absorbance at 450 nm was determined.

As a result, as shown in Table 6 it was confirmed that when the monoclonal antibody originating from hybridoma AMGC1 was used, this antibody can react with ribosomal proteins L7/L12 of all bacteria such as *Neisseria gonorrhoeae*, *Haemophilus influenzae*, and *Streptococcus pneumoniae*.

**Table 6**

| Results of detection of AMGC1 antibody and Ribosomal Protein L7/L12 of microorganisms | |
|---|---|
| | |
| *Neisseria gonorrhoeae* | + |
| *Haemophilus influenzae* | + |
| *Streptococcus pneumoniae* | + |
| (+: Positive) | |

The AMGC1 antibody obtained here is very useful as an antibody used for the detection of microorganisms in the so-called sandwich assay by optical immunoassay and ELISA method, in combination with an anti-ribosomal protein L7/L12 antibody which is specific to each microorganism.

### Industrial Applicability

According to the present invention, not only microorganisms can be detected specifically according to species, but also microorganisms of all serotypes in the same species can be detected at a high precision, by using antibodies to intracellular molecules of the same function.

By using antibodies to ribosomal proteins L7/L12 of various microorganisms as such antibodies, *Haemophilus influenzae* and *Neisseria gonorrhoeae* can be detected precisely.

Moreover, specific antibodies used for the detection of various kinds of microorganisms can be prepared by using intracellular molecules exhibiting same functions in various microorganisms as an antigen.

### REMARKS TO DEPOSITED MICROORGANISMS

The organization in which the microorganisms have been deposited:
National Institute of Bioscience and Human
Technology, the Agency of Industrial Science and Technology
Address: 1-1-3, Higashi, Yatabe-cho, Tsukuba-shi, Ibaraki-ken, Japan (Postal Code: 305).
Date of deposition: July 28, 1999
Number of deposition given by the deposition organization: FERM BP-6807

## Claims

1. Antibodies directed to ribosomal protein L7/L12 of bacteria which react specifically with ribosomal protein L7/L12 of bacteria from which the ribosomal protein L7/L12 is isolated and which can distinguish said bacteria genus or species from other bacteria genus or species, respectively.

2. The antibodies according to claim 1, where said bacteria are bacteria which cause a sexually transmitted disease (STD).

3. The antibodies according to claim 1, where said bacteria are bacteria which cause respiratory tract infection.

4. The antibody according to claim 3, where the causative bacteria of respiratory tract infection are bacteria of *Haemophilus influenzae.*

5. The antibody according to claim 3, where the causative bacteria of respiratory tract infection are bacteria of *Streptococcus pneumoniae*.

6. The antibody according to claim 3, where the causative bacteria of respiratory tract infection are bacteria of *Chlamydia pneumoniae.*

7. The antibody according to claim 3, where the causative bacteria of respiratory tract infection are bacteria of *Mycoplasma pneumoniae.*

8. The antibody according to claim 2, where the causative bacteria of sexually transmitted diseases (STD) are bacteria of *Neisseria gonorrhoeae*.

9. The antibody according to claim 8, which is the antibody to ribosomal protein L7/L12 of *Neisseria gonorrhoeae* and which recognizes a continuous amino acid sequence moiety from 5 to 30 amino acids including the 115^{th} alanine in the amino acid sequence of Sequence ID No. 22 of the Sequence Table.

10. A method of detecting bacteria, which is **characterized by** the fact that any antibody according to claims 1 to 9 is used.

11. A reagent kit for detecting bacteria which is **characterized by** comprising an antibody according to any of claims 1 to 9.

12. A method of preparing any antibody described in any one of the claims 1 to 9, **characterized by** the fact that ribosomal protein L7/L12 of bacteria obtained by a gene manipulation procedure or by isolation from bacteria, or a peptide moiety thereof, or a synthesized peptide corresponding to the peptide moiety is used as an immunogen.

## Patentansprüche

1. Antikörper, die auf das ribosomale Protein L7/L12 von Bakterien gerichtet sind, welche spezifisch mit dem ribosomalen Protein L7/L12 von Bakterien reagieren, aus denen das ribosomale Protein L7/L12 isoliert wird, und welche die Bakteriengattungen oder Spezies von anderen Bakteriengattungen beziehungsweise Spezies unterscheiden können.

2. Antikörper gemäß Anspruch 1, wobei die Bakterien Bakterien sind, die eine geschlechtlich übertragbare Krankheit (STD) verursachen.

3. Antikörper gemäß Anspruch 1, wobei die Bakterien Bakterien sind, die eine Infektion der Atemwege verursachen.

4. Antikörper gemäß Anspruch 3, wobei die Bakterien, die die Infektion der Atemwege verursachen, Bakterien von *Haemophilius influenzae* sind.

5. Antikörper gemäß Anspruch 3, wobei die Bakterien, die die Infektion der Atemwege verursachen, Bakterien von Streptococcus pneumoniae sind.

6. Antikörper gemäß Anspruch 3, wobei die Bakterien, die die Infektion der Atemwege verursachen, Bakterien von *Chlamydia pneumoniae* sind.

7. Antikörper gemäß Anspruch 3, wobei die Bakterien, die die Infektion der Atemwege verursachen, Bakterien von *Mycoplasma pneumoniae* sind.

8. Antikörper gemäß Anspruch 2, wobei die Bakterien, die die geschlechtlich übertragbaren Krankheiten (STD) verursachen, Bakterien von *Neisseria gonorrhoeae* sind.

9. Antikörper gemäß Anspruch 8, welcher der Antikörper für das ribosomale Protein L7/L12 von *Neisseria gonorrhoeae* ist und der eine zusammenhängende Aminosäuresequenz-Einheit von 5 bis 30 Aminosäuren erkennt, die das 115. Alanin in der Aminosäuresequenz von Sequenz ID No. 22 der Sequenzliste enthält.

10. Verfahren zum Nachweisen von Bakterien, **dadurch gekennzeichnet, dass** irgendein Antikörper gemäß den Ansprüchen 1 bis 9 verwendet wird.

11. Kit von Reagenzien zum Nachweis von Bakterien, **dadurch gekennzeichnet, dass** es einen Antikörper gemäß einem der Ansprüche 1 bis 9 umfasst.

12. Verfahren zur Herstellung irgendeines Antikörpers, der in irgendeinem der Ansprüche 1 bis 9 beschrieben ist, **dadurch gekennzeichnet, dass** ribosomales Protein L7/L12 von Bakterien, erhalten durch ein Genmanipulationsverfahren oder durch Isolierung aus Bakterien, oder eine Peptideinheit davon, oder ein synthetisiertes Peptid, das der Peptideinheit entspricht, als ein Immunogen verwendet wird.

## Revendications

1. Anticorps dirigés contre la protéine ribosomique L7/L12 de bactéries, qui réagissent spécifiquement avec la protéine ribosomique L7/L12 de bactéries, desquelles la protéine ribosomique L7/L12 est isolée et qui peuvent distinguer le genre ou l'espèce de bactérie d'autres genres ou espèces de bactéries respectivement.

2. Anticorps suivant la revendication 1, dans lequel les bactéries sont des bactéries qui provoquent une maladie transmissible sexuellement (MTS).

3. Anticorps suivant la revendication 1, dans lequel les bactéries sont des bactéries qui provoquent une infection des voies respiratoires.

4. Anticorps suivant la revendication 3, dans lequel les bactéries qui provoquent une infection des voies respiratoires sont des bactéries d'Haemophilus influenzae.

5. Anticorps suivant la revendication 3, dans lequel les bactéries qui provoquent une infection des voies respiratoires sont des bactéries de Streptococcus pneumoniae.

6. Anticorps suivant la revendication 3, dans lequel les bactéries qui provoquent une infection des voies respiratoires sont des bactéries de Chlamydia pneumoniae.

7. Anticorps suivant la revendication 3, dans lequel les bactéries qui provoquent une infection des voies respiratoires sont des bactéries de Mycoplasma pneumoniae.

8. Anticorps suivant la revendication 2, dans lequel les bactéries qui provoquent des maladies transmissibles sexuellement (MTS) sont des bactéries de Neisseria gonorrhoeae.

9. Anticorps suivant la revendication 8, qui est l'anticorps dirigé contre la protéine ribosomique L7/L12 de Neisseria gonorrhoeae et qui reconnaît un fragment continu d'une séquence d'acides aminés ayant de 5 à 30 acides aminés comprenant la 115ème alanine de la séquence d'acides aminés de la séquence ayant le numéro d'identification 22 du tableau des séquences.

10. Procédé de détection de bactéries, qui est **caractérisé par le fait que** l'on utilise l'un quelconque des anticorps suivant les revendications 1 à 9.

11. Trousse de réactif pour détecter des bactéries, qui est **caractérisée en ce qu'**elle comprend un anticorps suivant l'une quelconque des revendications 1 à 9.

12. Procédé de préparation de l'un quelconque des anticorps décrit à l'une quelconque des revendications 1 à 9, **caractérisé par le fait que** l'on utilise comme immunogène de la protéine ribosomique L7/L12 de bactérie obtenue par manipulation génétique ou par isolation à partir d'une bactérie ou de son fragment de peptides ou un peptide synthétisé correspondant au fragment de peptides.


### SEQUENCE LISTING

<110> ASAHIKASEI KOGYO KABUSIKI KAISHA

<120> Antibodies for Detecting Microorganisms

<130> ASAHI-2

<150> JP 10/230204
<151> 1998-7-31

<160> 22

<210> 1
<211> 369
<212> DNA
<213> Haemophilus influenzae

<400> 1

<2T0> 2
<211> 123
<212> PRT
<213> Haemophilus influenzae

<400> 2

<210> 3
<211> 375
<212> DNA
<213> Helicobacter pylori

<400> 3
   atg gca att tca aaa gaa gaa gtg tta gag tat att ggt tca ttg agc 48
   Met Ala Ile Ser Lys Glu Glu Val Leu Glu Tyr Ile Gly Ser Leu Ser
   1 5 10 15
   gtt tta gag ctt tct gaa ttg gtt aaa atg ttt gag gaa aaa ttt ggc 96
   Val Leu Glu Leu Ser Glu Leu Val Lys Met Phe Glu Glu Lys Phe Gly
   20 25 30
   gtg agc gcg act cca acg gtc gta gcg ggt gcg gct gta gct ggc ggt 144
   Val Ser Ala Thr Pro Thr Val Val Ala Gly Ala Ala Val Ala Gly Gly
   35 40 45
   gca gcg gct gag agc gaa gaa aaa acc gaa ttt aat gtg att ttg gcc 192
   Ala Ala Ala Glu Ser Glu Glu Lys Thr Glu Phe Asn Val Ile Leu Ala
   50 55 60
   gat agc ggt gct gaa aaa att aag gtg att aaa gtg gtt cgt gaa atc 240
   Asp Ser Gly Ala Glu Lys Ile Lys Val Ile Lys Val Val Arg Glu Ile
   65 70 75 80
   act gga ctt ggc ctg aaa gaa gct aaa gac gct acc gaa aaa acc cct 288
   Thr Gly Leu Gly Leu Lys Glu Ala Lys Asp Ala Thr Glu Lys Thr Pro
   85 90 95
   cat gtg ctt aaa gag ggc gtg aat aaa gaa gaa gct gaa acc atc aag 336
   His Val Leu Lys Glu Gly Val Asn Lys Glu Glu Ala Glu Thr Ile Lys
   100 105 110
   aag aaa ctt gaa gaa gta ggc gct aag gtt gaa gtc aag 375
   Lys Lys Leu Glu Glu Val Gly Ala Lys Val Glu Val Lys
   115 120 125

<210> 4
<211> 125
<212> PRT
<213> Helicobacter pylori

<400> 4
   Met Ala Ile Ser Lys Glu Glu Val Leu Glu Tyr Ile Gly Ser Leu Ser
   1               5                   10                  15
   Val Leu Glu Leu Ser Glu Leu Val Lys Met Phe Glu Glu Lys Phe Gly
               20                  25                  30
   Val Ser Ala Thr Pro Thr Val Val Ala Gly Ala Ala Val Ala Gly Gly
           35                  40                  45
   Ala Ala Ala Glu Ser Glu Glu Lys Thr Glu Phe Asn Val Ile Leu Ala
       50                  55                  60
   Asp Ser Gly Ala Glu Lys Ile Lys Val Ile Lys Val Val Arg Glu Ile
   65                  70                  75                  80
   Thr Gly Leu Gly Leu Lys Glu Ala Lys Asp Ala Thr Glu Lys Thr Pro
                   85                  90                  95
   His Val Leu Lys Glu Gly Val Asn Lys Glu Glu Ala Glu Thr Ile Lys
               100                 105                 110
   Lys Lys Leu Glu Glu Val Gly Ala Lys Val Glu Val Lys
           115                 120                 125

<210> 5
<211> 366
<212> DNA
<213> Streptococcus pneumoniae

<400> 5
   atg gca ttg aac att gaa aac att att gct gaa att aaa gaa gct tca 48
   Met Ala Leu Asn Ile Glu Asn Ile Ile Ala Glu Ile Lys Glu Ala Ser
   1 5 10 15
   atc ctt gaa ttg aac gac ctt gta aaa gct atc gaa gaa gaa ttt ggt 96
   Ile Leu Glu Leu Asn Asp Leu Val Lys Ala Ile Glu Glu Glu Phe Gly
   20 25 30
   gta act gca gct gct cct gta gct gtt gct gca gct gat gca gct gat 144
   Val Thr Ala Ala Ala Pro Val Ala Val Ala Ala Ala Asp Ala Ala Asp
   35 40 45
   gct ggt gct gct aaa gat tca ttc gac gtt gaa ttg aca tct gca ggc 192
   Ala Gly Ala Ala Lys Asp Ser Phe Asp Val Glu Leu Thr Ser Ala Gly
   50 55 60
   gac aaa aaa gtt ggc gtt atc aaa gtt gta cgt gaa atc act ggt ctt 240
   Asp Lys Lys Val Gly Val Ile Lys Val Val Arg Glu Ile Thr Gly Leu
   65 70 75 80
   ggt ctt aaa gaa gct aaa gaa ctt gtt gac ggt gca cca gca ctt gtt 288
   Gly Leu Lys Glu Ala Lys Glu Leu Val Asp Gly Ala Pro Ala Leu Val
   85 90 95
   aaa gaa ggc gtt gca act gca gaa gct gaa gaa atc aaa gct aaa ttg 336
   Lys Glu Gly Val Ala Thr Ala Glu Ala Glu Glu Ile Lys Ala Lys Leu
   100 105 110
   gaa gaa gct gga gct tca gtt act ctt aaa 366
   Glu Glu Ala Gly Ala Ser Val Thr Leu Lys
   115 120

<210> 6
<211> 122
<212> PRT
<213> Streptococcus pneumoniae

<400> 6
   Met Ala Leu Asn Ile Glu Asn Ile Ile Ala Glu Ile Lys Glu Ala Ser
   1 5 10 15
   Ile Leu Glu Leu Asn Asp Leu Val Lys Ala Ile Glu Glu Glu Phe Gly
   20 25 30
   Val Thr Ala Ala Ala Pro Val Ala Val Ala Ala Ala Asp Ala Ala Asp
   35 40 45
   Ala Gly Ala Ala Lys Asp Ser Phe Asp Val Glu Leu Thr Ser Ala Gly
   50 55 60
   Asp Lys Lys Val Gly Val Ile Lys Val Val Arg Glu Ile Thr Gly Leu
   65 70 75 80
   Gly Leu Lys Glu Ala Lys Glu Leu Val Asp Gly Ala Pro Ala Leu Val
   85 90 95
   Lys Glu Gly Val Ala Thr Ala Glu Ala Glu Glu Ile Lys Ala Lys Leu
   100 105 110
   Glu Glu Ala Gly Ala Ser Val Thr Leu Lys
   115 120

<210> 7
<211> 369
<212> DNA
<213> Neisseria gonorrhoeae

<400>7
   atg gct att act aaa gaa gac att ttg gaa gca gtt ggt tct ttg acc 48
   Met Ala Ile Thr Lys Glu Asp Ile Leu Glu Ala Val Gly Ser Leu Thr
   1 5 10 15
   gta atg gaa ttg aat gac ctg gtt aaa gct ttt gaa gaa aaa ttc ggt 96
   Val Met Glu Leu Asn Asp Leu Val Lys Ala Phe Glu Glu Lys Phe Gly
   20 25 30
   gtt tct gct gct gct gtt gca gtt gca ggt cct gct ggt gcc ggt gct 144
   Val Ser Ala Ala Ala Val Ala Val Ala Gly Pro Ala Gly Ala Gly Ala
   35 40 45
   gcc gat gct gaa gaa aaa acc gaa ttt gat gtc gtt ttg gct tct gcc 192
   Ala Asp Ala Glu Glu Lys Thr Glu Phe Asp Val Val Leu Ala Ser Ala
   50 55 60
   ggc gat caa aaa gtc ggc gtg att aaa gtt gtc cgt gca att act ggt 240
   Gly Asp Gln Lys Val Gly Val Ile Lys Val Val Arg Ala Ile Thr Gly
   65 70 75 80
   ttg ggt ctg aaa gaa gct aaa gac atc gtt gac ggc gca cct aaa acc 288
   Leu Gly Leu Lys Glu Ala Lys Asp Ile Val Asp Gly Ala Pro Lys Thr
   85 90 95
   att aaa gag ggt gtt tct aaa gct gaa gcc gaa gac atc caa aaa caa 336
   Ile Lys Glu Gly Val Ser Lys Ala Glu Ala Glu Asp Ile Gln Lys Gln
   100 105 110
   ctg gaa gca gca ggc gct aaa gtc gaa atc aaa 369
   Leu Glu Ala Ala Gly Ala Lys Val Glu Ile Lys
   115 120

<210> 8
<211> 123
<212> PRT
<213> Neisseria gonorrhoeae

<400> 8
   Met Ala Ile Thr Lys Glu Asp Ile Leu Glu Ala Val Gly Ser Leu Thr
   1 5 10 15
   Val Met Glu Leu Asn Asp Leu Val Lys Ala Phe Glu Glu Lys Phe Gly
   20 25 30
   Val Ser Ala Ala Ala Val Ala Val Ala Gly Pro Ala Gly Ala Gly Ala
   35 40 45
   Ala Asp Ala Glu Glu Lys Thr Glu Phe Asp Val Val Leu Ala Ser Ala
   50 55 60
   Gly Asp Gln Lys Val Gly Val Ile Lys Val Val Arg Ala Ile Thr Gly
   65 70 75 80
   Leu Gly Leu Lys Glu Ala Lys Asp Ile Val Asp Gly Ala Pro Lys Thr
   85 90 95
   Ile Lys Glu Gly Val Ser Lys Ala Glu Ala Glu Asp Ile Gln Lys Gln
   100 105 110
   Leu Glu Ala Ala Gly Ala Lys Val Glu Ile Lys
   115 120

<210> 9
<211> 369
<212> DNA
<213> Neisseria meningitidis

<400> 9
   atg gct att act aaa gaa gac att ttg gaa gca gtt ggt tct ttg acc 48
   Met Ala Ile Thr Lys Glu Asp Ile Leu Glu Ala Val Gly Ser Leu Thr
   1 5 10 15
   gta atg gaa ttg aac gac ttg gtt aaa gct ttt gaa gaa aaa ttc ggt 96
   Val Met Glu Leu Asn Asp Leu Val Lys Ala Phe Glu Glu Lys Phe Gly
   20 25 30
   gtt tct gct gct gct gtt gca gtt gca ggt cct gct ggt gcc ggt gct 144
   Val Ser Ala Ala Ala Val Ala Val Ala Gly Pro Ala Gly Ala Gly Ala
   35 40 45
   gcc gat gct gaa gaa aaa acc gaa ttt gat gtc gtt ttg gct tct gcc 192
   Ala Asp Ala Glu Glu Lys Thr Glu Phe Asp Val Val Leu Ala Ser Ala
   50 55 60
   ggt gat caa aaa gtc ggc gtg att aaa gtt gtc cgt gca att acc ggt 240
   Gly Asp Gln Lys Val Gly Val Ile Lys Val Val Arg Ala Ile Thr Gly
   65 70 75 80
   ttg ggt ctg aaa gaa gct aaa gac atc gtt gac ggt gca cct aaa acc 288
   Leu Gly Leu Lys Glu Ala Lys Asp Ile Val Asp Gly Ala Pro Lys Thr
   85 90 95
   att aaa gag ggt gtt tct aaa gct gaa gcc gaa gac atc caa aaa caa 336
   Ile Lys Glu Gly Val Ser Lys Ala Glu Ala Glu Asp Ile Gln Lys Gln
   100 105 110
   ctg gaa gaa gcc ggc gct aaa gtc gaa atc aaa 369
   Leu Glu Glu Ala Gly Ala Lys Val Glu Ile Lys
   115 120

<210> 10
<211> 123
<212> PRT
<213> Neisseria menigitidis

<400> 10
   Met Ala Ile Thr Lys Glu Asp Ile Leu Glu Ala Val Gly Ser Leu Thr
   1 5 10 15
   Val Met Glu Leu Asn Asp Leu Val Lys Ala Phe Glu Glu Lys Phe Gly
   20 25 30
   Val Ser Ala Ala Ala Val Ala Val Ala Gly Pro Ala Gly Ala Gly Ala
   35 40 45
   Ala Asp Ala Glu Glu Lys Thr Glu Phe Asp Val Val Leu Ala Ser Ala
   50 55 60
   Gly Asp Gln Lys Val Gly Val Ile Lys Val Val Arg Ala Ile Thr Gly
   65 70 75 80
   Leu Gly Leu Lys Glu Ala Lys Asp Ile Val Asp Gly Ala Pro Lys Thr
   85 90 95
   Ile Lys Glu Gly Val Ser Lys Ala Glu Ala Glu Asp Ile Gln Lys Gln
   100 105 110
   Leu Glu Glu Ala Gly Ala Lys Val Glu Ile Lys
   115 120

<210> 11
<211> 31
<212> DNA
<213> artificial sequence

<220>
<223> the primers DNA for PCR used to acquire the ribosomal protein L7/L12 gene from H. influenzae.

<400> 11
   gtaaggatcc atgtcattaa ctaacgaaca a 31

<210> 12
<211> 34
<212> DNA
<213> artificial sequence

<220>
<223> The primers DNA for PCR used to acquire the ribosomal protein L7/L12 gene from H. influenzae.

<400> 12
   agcatctcga gatttaactt ctacttctgc accc 34

<210> 13
<211> 33
<212> DNA
<213> artificial sequence

<220>
<223> The primers DNA for PCR used to acquire the ribosomal protein L7/L12 gene from S. pneumoniae.

<400> 13
   ggaaggatcc atggcattga acattgaaaa cat 33

<210> 14
<211>33
<212> DNA
<213> artificial sequence

<220>
<223> The primers DNA for PCR used to acquire the ribosomal protein L7/L12 gene from S. pneumoniae.

<400> 14
   tactctcgag tttaagagta actgaagctc cag 33

<210> 15
<211> 31
<212> DNA
<213> artificial sequence

<220>
<223> The primers DNA for PCR used to acquire the ribosomal protein L7/L12 gene from N. gonorrhoeae.

<400> 15
   gtaaggatcc atggctatta ctaaagaaga c 31

<210> 16
<211> 34
<212> DNA
<213> artificial sequence

<220>
<223> The primers DNA for PCR used to acquire the ribosomal protein L7/L12 gene from N. gonorrhoeae.

<400> 16
   agcatctcga gatttgattt cgactttagc gcct 34

<210> 17
<211> 369
<212> DNA
<213> Haemophilus influenzae

<400> 17
   atg tca tta act aac gaa caa atc att gaa gcg att gct tca aaa act 48
   Met Ser Leu Thr Asn Glu Gln Ile Ile Glu Ala Ile Ala Ser Lys Thr
   1 5 10 15
   gta act gaa atc gtt gaa tta atc gca gcg atg gaa gaa aaa ttc ggt 96
   Val Thr Glu Ile Val Glu Leu Ile Ala Ala Met Glu Glu Lys Phe Gly
   20 25 30
   gtt tca gca gcg gca gca gta gca gca gct cca gca gca ggc ggt gca 144
   Val Ser Ala Ala Ala Ala Val Ala Ala Ala Pro Ala Ala Gly Gly Ala
   35 40 45
   gcg gca gca gaa gaa aaa act gaa ttc gac gtt gta ctt aaa tct gca 192
   Ala Ala Ala Glu Glu Lys Thr Glu Phe Asp Val Val Leu Lys Ser Ala
   50 55 60
   ggt gcg aac aaa gta gca gta att aaa gca gta cgt ggt gca act ggt 240
   Gly Ala Asn Lys Val Ala Val Ile Lys Ala Val Arg Gly Ala Thr Gly
   65 70 75 80
   tta ggc tta aaa gaa gct aaa gat tta gtt gaa tct gct cca gct aac 288
   Leu Gly Leu Lys Glu Ala Lys Asp Leu Val Glu Ser Ala Pro Ala Asn
   85 90 95
   tta aaa gaa ggc gtt tct aaa gaa gaa gct gaa gca ctt aag aaa gaa 336
   Leu Lys Glu Gly Val Ser Lys Glu Glu Ala Glu Ala Leu Lys Lys Glu
   100 105 110
   tta gaa gaa gcg ggt gca gaa gta gaa gtt aaa 369
   Leu Glu Glu Ala Gly Ala Glu Val Glu Val Lys
   115 120

<210> 18
<211> 123
<212> PRT
<213> Haemophilus influenzae

<400> 18
   Met Ser Leu Thr Asn Glu Gln Ile Ile Glu Ala Ile Ala Ser Lys Thr
   1 5 10 15
   Val Thr Glu Ile Val Glu Leu Ile Ala Ala Met Glu Glu Lys Phe Gly
   20 25 30
   Val Ser Ala Ala Ala Ala Val Ala Ala Ala Pro Ala Ala Gly Gly Ala
   35 40 45
   Ala Ala Ala Glu Glu Lys Thr Glu Phe Asp Val Val Leu Lys Ser Ala
   50 55 60
   Gly Ala Asn Lys Val Ala Val Ile Lys Ala Val Arg Gly Ala Thr Gly
   65 70 75 80
   Leu Gly Leu Lys Glu Ala Lys Asp Leu Val Glu Ser Ala Pro Ala Asn
   85 90 95
   Leu Lys Glu Gly Val Ser Lys Glu Glu Ala Glu Ala Leu Lys Lys Glu
   100 105 110
   Leu Glu Glu Ala Gly Ala Glu Val Glu Val Lys
   115 120

<210> 19
<211> 366
<212> DNA
<213> Streptococcus pneumoniae

<400> 19
   atg gca ttg aac att gaa aac att att gct gaa att aaa gaa gct tca 48
   Met Ala Leu Asn Ile Glu Asn Ile Ile Ala Glu Ile Lys Glu Ala Ser
   1 5 10 15
   atc ctt gaa ttg aac gac ctt gta aaa gct atc gaa gaa gaa ttt ggt 96
   Ile Leu Glu Leu Asn Asp Leu Val Lys Ala Ile Glu Glu Glu Phe Gly
   20 25 30
   gta act gca gct gct cct gta gct gtt gct gca gct gat gca gct gat 144
   Val Thr Ala Ala Ala Pro Val Ala Val Ala Ala Ala Asp Ala Ala Asp
   35 40 45
   gct ggt gct gct aaa gat tca ttc gac gtt gaa ttg aca tct gca ggc 192
   Ala Gly Ala Ala Lys Asp Ser Phe Asp Val Glu Leu Thr Ser Ala Gly
   50 55 60
   gac aaa aaa gtt ggc gtt atc aaa gtt gta cgt gaa atc act ggt ctt 240
   Asp Lys Lys Val Gly Val Ile Lys Val Val Arg Glu Ile Thr Gly Leu
   65 70 75 80
   ggt ctt aaa gaa gct aaa gaa ctt gtt gac ggt gca cca gca ctt gtt 288
   Gly Leu Lys Glu Ala Lys Glu Leu Val Asp Gly Ala Pro Ala Leu Val
   85 90 95
   aaa gaa ggc gtt gca act gca gaa gct gaa gaa atc aaa gct aaa ttg 336
   Lys Glu Gly Val Ala Thr Ala Glu Ala Glu Glu Ile Lys Ala Lys Leu
   100 105 110
   gaa gaa gct gga gct tca gtt act ctt aaa 366
   Glu Glu Ala Gly Ala Ser Val Thr Leu Lys
   115 120

<210> 20
<211> 122
<212> PRT
<213> Streptococcus pneumoniae

<400> 20
   Met Ala Leu Asn Ile Glu Asn Ile Ile Ala Glu Ile Lys Glu Ala Ser
   1 5 10 15
   Ile Leu Glu Leu Asn Asp Leu Val Lys Ala Ile Glu Glu Glu Phe Gly
   20 25 30
   Val Thr Ala Ala Ala Pro Val Ala Val Ala Ala Ala Asp Ala Ala Asp
   35 40 45
   Ala Gly Ala Ala Lys Asp Ser Phe Asp Val Glu Leu Thr Ser Ala Gly
   50 55 60
   Asp Lys Lys Val Gly Val Ile Lys Val Val Arg Glu Ile Thr Gly Leu
   65 70 75 80
   Gly Leu Lys Glu Ala Lys Glu Leu Val Asp Gly Ala Pro Ala Leu Val
   85 90 95
   Lys Glu Gly Val Ala Thr Ala Glu Ala Glu Glu Ile Lys Ala Lys Leu
   100 105 110
   Glu Glu Ala Gly Ala Ser Val Thr Leu Lys
   115 120

<210> 21
<211> 369
<212> DNA
<213> Neisseria gonorrhoeae

<400> 21
   atg gct att act aaa gaa gac att ttg gaa gca gtt ggt tct ttg acc 48
   Met Ala Ile Thr Lys Glu Asp Ile Leu Glu Ala Val Gly Ser Leu Thr
   1 5 10 15
   gta atg gaa ttg aat gac ctg gtt aaa gct ttt gaa gaa aaa ttc ggt 96
   Val Met Glu Leu Asn Asp Leu Val Lys Ala Phe Glu Glu Lys Phe Gly
   20 25 30
   gtt tct gct gct gct gtt gca gtt gca ggt cct gct ggt gcc ggt gct 144
   Val Ser Ala Ala Ala Val Ala Val Ala Gly Pro Ala Gly Ala Gly Ala
   35 40 45
   gcc gat gct gaa gaa aaa acc gaa ttt gat gtc gtt ttg gct tct gcc 192
   Ala Asp Ala Glu Glu Lys Thr Glu Phe Asp Val Val Leu Ala Ser Ala
   50 55 60
   ggc gat caa aaa gtc ggc gtg att aaa gtt gtc cgt gca att act ggt 240
   Gly Asp Gln Lys Val Gly Val Ile Lys Val Val Arg Ala Ile Thr Gly
   65 70 75 80
   ttg ggt ctg aaa gaa gct aaa gac atc gtt gac ggc gca cct aaa acc 288
   Leu Gly Leu Lys Glu Ala Lys Asp Ile Val Asp Gly Ala Pro Lys Thr
   85 90 95
   att aaa gag ggt gtt tct aaa gct gaa gcc gaa gac atc caa aaa caa 336
   Ile Lys Glu Gly Val Ser Lys Ala Glu Ala Glu Asp Ile Gln Lys Gln
   100 105 110
   ctg gaa gca gca ggc gct aaa gtc gaa atc aaa 369
   Leu Glu Ala Ala Gly Ala Lys Val Glu Ile Lys
   115 120

<210> 22
<211> 123
<212> PRT
<213> Neisseria gonorrhoeae

<400> 22
   Met Ala Ile Thr Lys Glu Asp Ile Leu Glu Ala Val Gly Ser Leu Thr
   1 5 10 15
   Val Met Glu Leu Asn Asp Leu Val Lys Ala Phe Glu Glu Lys Phe Gly
   20 25 30
   Val Ser Ala Ala Ala Val Ala Val Ala Gly Pro Ala Gly Ala Gly Ala
   35 40 45
   Ala Asp Ala Glu Glu Lys Thr Glu Phe Asp Val Val Leu Ala Ser Ala
   50 55 60
   Gly Asp Gln Lys Val Gly Val Ile Lys Val Val Arg Ala Ile Thr Gly
   65 70 75 80
   Leu Gly Leu Lys Glu Ala Lys Asp Ile Val Asp Gly Ala Pro Lys Thr
   85 90 95
   Ile Lys Glu Gly Val Ser Lys Ala Glu Ala Glu Asp Ile Gln Lys Gln
   100 105 110
   Leu Glu Ala Ala Gly Ala Lys Val Glu Ile Lys
   115 120